# EUROPEAN PATENT APPLICATION

(11) **EP 4 834 947 A1**
(43) Date of publication of application: **04.11.2026**
(21) Application number: 24910910.9
(22) Date of filing: 19.12.2024
(51) Int. Cl.: C07K 19/00, C07K 14/705, C12N 15/85, C12N 5/0783

(54) **CHIMERIC ANTIGEN RECEPTOR TARGETING LY6G6D AND USE THEREOF**

(30) Priority: 26.12.2023 CN 202311797447; 26.12.2023 CN 202311797256
(71) Applicant: Sunshine Lake Pharma Co., Ltd., Dongguan, Guangdong 523000 (CN)
(72) Inventor: CHEN, Shiyou, Dongguan, Guangdong 523871 (CN); YANG, Xiaodan, Dongguan, Guangdong 523871 (CN); LI, Lijia, Dongguan, Guangdong 523871 (CN); TAN, Xiumei, Dongguan, Guangdong 523871 (CN); ZHAO, Guanghui, Dongguan, Guangdong 523871 (CN); SUN, Dongshan, Dongguan, Guangdong 523871 (CN); ZHANG, Kuo, Dongguan, Guangdong 523871 (CN); WAN, Dan, Dongguan, Guangdong 523871 (CN); KANG, Ning, Dongguan, Guangdong 523871 (CN); LI, Xiaoping, Dongguan, Guangdong 523871 (CN)
(74) Representative: Hoffmann Eitle
(86) International application number: PCT/CN2024/140478
(87) International publication number: WO 2025/139979

(57) **Abstract**

The present invention provides a chimeric antigen receptor targeting Ly6G6D and a use thereof. Specifically, the present invention provides a CAR that specifically binds to Ly6G6D, comprising a Ly6G6D-binding domain, a transmembrane domain, a costimulatory domain, and an intracellular signaling domain. The present invention further provides a use of the CAR for treating a disease or disorder associated with the expression of Ly6G6D.

## Description

### FIELD OF THE INVENTION

The subject matter disclosed in the present invention provides methods and compositions for treating cancer. Specifically, the present invention provides a chimeric antigen receptor targeting Ly6G6D, immune effector cells comprising the chimeric antigen receptor, and methods of using these immune effector cells for treating cancer.

### BACKGROUND ART

Cell-based immunotherapy is a therapeutic approach with potential for treating cancer. T cells and other immune cells can be modified to target tumor antigens by introducing genetic material encoding artificial or synthetic receptors against the antigens, which are called chimeric antigen receptors (CARs) and are specific for the selected antigens. Targeted T cell therapy using CARs has achieved clinical success in treating cancer.

Lymphocyte antigen 6 family member G6D (Ly6G6D) is a leukocyte antigen cluster located in the major histocompatibility complex (MHC) class III region on chromosome 6, encoding a protein of 133 amino acid residues with a molecular weight of approximately 13.7 kDa. Like most family members, Ly6G6D is attached to the cell membrane via a glycosylphosphatidylinositol (GPI) anchor. Multiple studies have shown that lymphocyte antigen 6 family members play important roles in regulating immune, nervous system, and complement functions. Especially in cancer, many members of the lymphocyte antigen 6 family play critical roles in many types of cancer, such as gastric cancer, cervical cancer, breast cancer, ovarian cancer, lung cancer, and bladder cancer. Therefore, the lymphocyte antigen 6 gene family may play an important role in clinical practice, not only as a marker of disease prognosis but also as an important target for the development of new drugs. In-depth research on their biological functions will be of great significance for elucidating the functions of the lymphocyte antigen 6 family, analyzing protein interactions in their structures, exploring disease pathogenesis, and discovering new therapeutic targets.

Colorectal cancer can be classified into mismatch repair-deficient (dMMR) and mismatch repair-proficient (pMMR) types, wherein the dMMR type mainly includes microsatellite instability-high (MSI-H) subtype, and the pMMR type mainly includes microsatellite instability-low (MSI-L) and microsatellite stable (MSS) subtypes. For metastatic colorectal cancer of the MSI-H type, immune checkpoint inhibitors have shown good therapeutic efficacy; however, for the MSI-L and MSS types, which account for approximately 85% of all metastatic colorectal cancer patients, the response rate to immune checkpoint inhibitors is low, and patient survival is poor, creating an urgent need for more effective and safer therapeutic drugs. Studies have shown that Ly6G6D is highly expressed in pMMR-type advanced colorectal cancer tissues and is an ideal tumor-specific antigen.

### SUMMARY OF THE INVENTION

The present invention provides a chimeric antigen receptor targeting Ly6G6D, immune effector cells comprising the chimeric antigen receptor, and uses of using these immune effector cells for treating cancer.

Specifically, in one aspect, the present invention provides a chimeric antigen receptor targeting Ly6G6D, the chimeric antigen receptor comprises an extracellular antigen-binding domain that binds to Ly6G6D, a transmembrane domain and an intracellular domain, wherein the extracellular antigen-binding domain comprises an antibody or antigen-binding fragment that targets and binds to Ly6G6D, wherein the antibody or antigen-binding fragment comprises a heavy chain variable region, the heavy chain variable region comprises complementarity determining regions: H-CDR1, H-CDR2 and H-CDR3; wherein,
H-CDR1 has the amino acid sequence shown in SEQ ID NO: 1, SEQ ID NO: 7, SEQ ID NO: 13, SEQ ID NO: 18, SEQ ID NO: 22, SEQ ID NO: 28, SEQ ID NO: 34, SEQ ID NO: 40, SEQ ID NO: 46, SEQ ID NO: 50, SEQ ID NO: 127 or SEQ ID NO: 138; and
H-CDR2 has the amino acid sequence shown in SEQ ID NO: 2, SEQ ID NO: 8, SEQ ID NO: 14, SEQ ID NO: 19, SEQ ID NO: 23, SEQ ID NO: 29, SEQ ID NO: 35, SEQ ID NO: 41, SEQ ID NO: 47, SEQ ID NO: 51 or SEQ ID NO: 139; and
H-CDR3 has the amino acid sequence shown in SEQ ID NO: 3, SEQ ID NO: 9, SEQ ID NO: 15, SEQ ID NO: 20, SEQ ID NO: 24, SEQ ID NO: 30, SEQ ID NO: 36, SEQ ID NO: 42, SEQ ID NO: 48, SEQ ID NO: 52, SEQ ID NO: 128, SEQ ID NO: 132 or SEQ ID NO: 140;
   and the antibody or antigen-binding fragment comprises a light chain variable region, the light chain variable region comprises complementarity determining regions: L-CDR1, L-CDR2 and L-CDR3; wherein,
L-CDR1 has the amino acid sequence shown in SEQ ID NO: 4, SEQ ID NO: 10, SEQ ID NO: 25, SEQ ID NO: 31, SEQ ID NO: 37, SEQ ID NO: 43, SEQ ID NO: 53, SEQ ID NO: 129, SEQ ID NO: 135 or SEQ ID NO: 141; and
L-CDR2 has the amino acid sequence shown in SEQ ID NO: 5, SEQ ID NO: 11, SEQ ID NO: 16, SEQ ID NO: 26, SEQ ID NO: 32, SEQ ID NO: 38, SEQ ID NO: 44, SEQ ID NO: 54, SEQ ID NO: 130, SEQ ID NO: 133, SEQ ID NO: 136 or SEQ ID NO: 142; and
L-CDR3 has the amino acid sequence shown in SEQ ID NO: 6, SEQ ID NO: 12, SEQ ID NO: 17, SEQ ID NO: 21, SEQ ID NO: 27, SEQ ID NO: 33, SEQ ID NO: 39, SEQ ID NO: 45, SEQ ID NO: 49, SEQ ID NO: 131, SEQ ID NO: 134, SEQ ID NO: 137 or SEQ ID NO: 143.
NYAMH (SEQ ID NO: 1).
VISYDGSKKYYADSVKG (SEQ ID NO: 2).
PAGSTSGFDY (SEQ ID NO: 3).
TGTSSDVGGYNYVS (SEQ ID NO: 4).
DVSNRPS (SEQ ID NO: 5).
SSYTSSSTLVV (SEQ ID NO: 6).
DYGMS (SEQ ID NO: 7).
GINWNGGSTGYADSVKG (SEQ ID NO: 8).
SSVKHY (SEQ ID NO: 9).
SGSSSNIGTNTVN (SEQ ID NO: 10).
RNDLRPS (SEQ ID NO: 11).
AAWDDSLNGVV (SEQ ID NO: 12).
NYGIT (SEQ ID NO: 13).
WISPYNGHTIYAQKVQG (SEQ ID NO: 14).
GLNSRLQY (SEQ ID NO: 15).
DVSKRPS (SEQ ID NO: 16).
SSYTSSSTDV (SEQ ID NO: 17).
GYYMH (SEQ ID NO: 18).
WINPVSGGTNYAQKFQG (SEQ ID NO: 19).
DPFDI (SEQ ID NO: 20).
SSYTSRSTPGYV (SEQ ID NO: 21).
NYGFS (SEQ ID NO: 22).
WISSYNGNTKYAQKFRG (SEQ ID NO: 23).
GGDWPADY (SEQ ID NO: 24).
GLSSGSVSNNYYPS (SEQ ID NO: 25).
STNTRSS (SEQ ID NO: 26).
VVYMGSGIWV (SEQ ID NO: 27).
SYGIS (SEQ ID NO: 28).
WINPNSGGTNYAQKFQG (SEQ ID NO: 29).
AGDLRDYYYYGMDV (SEQ ID NO: 30).
TGSSSNIGAGYDVH (SEQ ID NO: 31).
GNSNRPS (SEQ ID NO: 32).
QSYDSSLSGWV (SEQ ID NO: 33).
NHFIH (SEQ ID NO: 34).
WISANSGNTDYAQKFQG (SEQ ID NO: 35).
DLATVDGFDI (SEQ ID NO: 36).
RSSQSLLHSNGYNYLD (SEQ ID NO: 37).
LGSNRAS (SEQ ID NO: 38).
MQALQTPLT (SEQ ID NO: 39).
SYSVS (SEQ ID NO: 40).
GIIPTFGRTNYAQKFQG (SEQ ID NO: 41).
VRKPYYGMDV (SEQ ID NO: 42).
RASQSIGRFLN (SEQ ID NO: 43).
GASRLQG (SEQ ID NO: 44).
QQSYSTPPT (SEQ ID NO: 45).
SYGIN (SEQ ID NO: 46).
WINTKTGNPTYVQGFTG (SEQ ID NO: 47).
DRNPYGMDV (SEQ ID NO: 48).
MQPLQALYT (SEQ ID NO: 49).
SYAIS (SEQ ID NO: 50).
GIIPIFGTANYAQKFQG (SEQ ID NO: 51).
TGGDYYYYGMDV (SEQ ID NO: 52).
RASQTISTYLN (SEQ ID NO: 53).
GASSLQT (SEQ ID NO: 54).
DYFLH (SEQ ID NO: 127).
DPFDY (SEQ ID NO: 128).
RSSQSLVHSDGNTYLS (SEQ ID NO: 129).
KISNRFS (SEQ ID NO: 130).
MQATQFPIT (SEQ ID NO: 131).
VGGSYYGGFDY (SEQ ID NO: 132).
LSSNRAS (SEQ ID NO: 133).
MQALQTPPT (SEQ ID NO: 134).
RSSQSLVYSDGNTYLS (SEQ ID NO: 135).
KVSNRFS (SEQ ID NO: 136).
MQATQYPLA (SEQ ID NO: 137).
NYWIG (SEQ ID NO: 138).
IIYPGDSDTRYSPSFQG (SEQ ID NO: 139).
PQGAFDI (SEQ ID NO: 140).
RSSQSLVYSDGNTYLN (SEQ ID NO: 141).
KVSNRDS (SEQ ID NO: 142).
MQGTHWPLT (SEQ ID NO: 143).

In some embodiments, complementarity determining regions of the heavy chain variable regions: H-CDR1, H-CDR2 and H-CDR3 are selected from any one of the following groups:
(1) H-CDR1 has the amino acid sequence shown in SEQ ID NO:1, H-CDR2 has the amino acid sequence shown in SEQ ID NO:2 and H-CDR3 has the amino acid sequence shown in SEQ ID NO:3; or
(2) H-CDR1 has the amino acid sequence shown in SEQ ID NO:7, H-CDR2 has the amino acid sequence shown in SEQ ID NO:8 and H-CDR3 has the amino acid sequence shown in SEQ ID NO:9; or
(3) H-CDR1 has the amino acid sequence shown in SEQ ID NO:13, H-CDR2 has the amino acid sequence shown in SEQ ID NO:14 and H-CDR3 has the amino acid sequence shown in SEQ ID NO:15; or
(4) H-CDR1 has the amino acid sequence shown in SEQ ID NO:18, H-CDR2 has the amino acid sequence shown in SEQ ID NO:19 and H-CDR3 has the amino acid sequence shown in SEQ ID NO:20; or
(5) H-CDR1 has the amino acid sequence shown in SEQ ID NO:22, H-CDR2 has the amino acid sequence shown in SEQ ID NO:23 and H-CDR3 has the amino acid sequence shown in SEQ ID NO:24; or
(6) H-CDR1 has the amino acid sequence shown in SEQ ID NO:28, H-CDR2 has the amino acid sequence shown in SEQ ID NO:29 and H-CDR3 has the amino acid sequence shown in SEQ ID NO:30; or
(7) H-CDR1 has the amino acid sequence shown in SEQ ID NO:34, H-CDR2 has the amino acid sequence shown in SEQ ID NO:35 and H-CDR3 has the amino acid sequence shown in SEQ ID NO:36; or
(8) H-CDR1 has the amino acid sequence shown in SEQ ID NO:40, H-CDR2 has the amino acid sequence shown in SEQ ID NO:41 and H-CDR3 has the amino acid sequence shown in SEQ ID NO:42; or
(9) H-CDR1 has the amino acid sequence shown in SEQ ID NO:46, H-CDR2 has the amino acid sequence shown in SEQ ID NO:47 and H-CDR3 has the amino acid sequence shown in SEQ ID NO:48; or
(10) H-CDR1 has the amino acid sequence shown in SEQ ID NO:50, H-CDR2 has the amino acid sequence shown in SEQ ID NO:51 and H-CDR3 has the amino acid sequence shown in SEQ ID NO:52 or
(11) H-CDR1 has the amino acid sequence shown in SEQ ID NO:127, H-CDR2 has the amino acid sequence shown in SEQ ID NO:29 and H-CDR3 has the amino acid sequence shown in SEQ ID NO:128; or
(12) H-CDR1 has the amino acid sequence shown in SEQ ID NO:18, H-CDR2 has the amino acid sequence shown in SEQ ID NO:29 and H-CDR3 has the amino acid sequence shown in SEQ ID NO:132; or
(13) H-CDR1 has the amino acid sequence shown in SEQ ID NO:18, H-CDR2 has the amino acid sequence shown in SEQ ID NO:29 and H-CDR3 has the amino acid sequence shown in SEQ ID NO:128; or
(14) H-CDR1 has the amino acid sequence shown in SEQ ID NO:138, H-CDR2 has the amino acid sequence shown in SEQ ID NO:139 and H-CDR3 has the amino acid sequence shown in SEQ ID NO: 140.

In some embodiments, complementarity determining regions of the light chain variable regions: L-CDR1, L-CDR2, and L-CDR3 are selected from any one of the following groups:
(1) L-CDR1 has the amino acid sequence shown in SEQ ID NO:4, L-CDR2 has the amino acid sequence shown in SEQ ID NO:5 and L-CDR3 has the amino acid sequence shown in SEQ ID NO:6; or
(2) L-CDR1 has the amino acid sequence shown in SEQ ID NO:10, L-CDR2 has the amino acid sequence shown in SEQ ID NO:11 and L-CDR3 has the amino acid sequence shown in SEQ ID NO:12; or
(3) L-CDR1 has the amino acid sequence shown in SEQ ID NO:4, L-CDR2 has the amino acid sequence shown in SEQ ID NO:16 and L-CDR3 has the amino acid sequence shown in SEQ ID NO:17; or
(4) L-CDR1 has the amino acid sequence shown in SEQ ID NO:4, L-CDR2 has the amino acid sequence shown in SEQ ID NO:16 and L-CDR3 has the amino acid sequence shown in SEQ ID NO:21; or
(5) L-CDR1 has the amino acid sequence shown in SEQ ID NO:25, L-CDR2 has the amino acid sequence shown in SEQ ID NO:26 and L-CDR3 has the amino acid sequence shown in SEQ ID NO:27; or
(6) L-CDR1 has the amino acid sequence shown in SEQ ID NO:31, L-CDR2 has the amino acid sequence shown in SEQ ID NO:32 and L-CDR3 has the amino acid sequence shown in SEQ ID NO:33; or
(7) L-CDR1 has the amino acid sequence shown in SEQ ID NO:37, L-CDR2 has the amino acid sequence shown in SEQ ID NO:38 and L-CDR3 has the amino acid sequence shown in SEQ ID NO:39; or
(8) L-CDR1 has the amino acid sequence shown in SEQ ID NO:43, L-CDR2 has the amino acid sequence shown in SEQ ID NO:44 and L-CDR3 has the amino acid sequence shown in SEQ ID NO:45; or
(9) L-CDR1 has the amino acid sequence shown in SEQ ID NO:37, L-CDR2 has the amino acid sequence shown in SEQ ID NO:38 and L-CDR3 has the amino acid sequence shown in SEQ ID NO:49; or
(10) L-CDR1 has the amino acid sequence shown in SEQ ID NO:53, L-CDR2 has the amino acid sequence shown in SEQ ID NO:54 and L-CDR3 has the amino acid sequence shown in SEQ ID NO:45; or
(11) L-CDR1 has the amino acid sequence shown in SEQ ID NO:129, L-CDR2 has the amino acid sequence shown in SEQ ID NO:130 and L-CDR3 has the amino acid sequence shown in SEQ ID NO:131; or
(12) L-CDR1 has the amino acid sequence shown in SEQ ID NO:37, L-CDR2 has the amino acid sequence shown in SEQ ID NO:133 and L-CDR3 has the amino acid sequence shown in SEQ ID NO:134; or
(13) L-CDR1 has the amino acid sequence shown in SEQ ID NO:135, L-CDR2 has the amino acid sequence shown in SEQ ID NO:136 and L-CDR3 has the amino acid sequence shown in SEQ ID NO:137; or
(14) L-CDR1 has the amino acid sequence shown in SEQ ID NO:141, L-CDR2 has the amino acid sequence shown in SEQ ID NO:142 and L-CDR3 has the amino acid sequence shown in SEQ ID NO: 143.

In some embodiments, the complementarity determining regions of the heavy chain variable regions: H-CDR1, H-CDR2, and H-CDR3, and the complementarity determining regions of the light chain variable regions: L-CDR1, L-CDR2, and L-CDR3 are selected from any one of the following groups:
(1) H-CDR1 has the amino acid sequence shown in SEQ ID NO:1, H-CDR2 has the amino acid sequence shown in SEQ ID NO:2 and H-CDR3 has the amino acid sequence shown in SEQ ID NO:3; and
   L-CDR1 has the amino acid sequence shown in SEQ ID NO:4, L-CDR2 has the amino acid sequence shown in SEQ ID NO:5 and L-CDR3 has the amino acid sequence shown in SEQ ID NO:6; or
(2) H-CDR1 has the amino acid sequence shown in SEQ ID NO:7, H-CDR2 has the amino acid sequence shown in SEQ ID NO:8 and H-CDR3 has the amino acid sequence shown in SEQ ID NO:9; and
   L-CDR1 has the amino acid sequence shown in SEQ ID NO:10, L-CDR2 has the amino acid sequence shown in SEQ ID NO:11 and L-CDR3 has the amino acid sequence shown in SEQ ID NO:12; or
(3) H-CDR1 has the amino acid sequence shown in SEQ ID NO:13, H-CDR2 has the amino acid sequence shown in SEQ ID NO:14 and H-CDR3 has the amino acid sequence shown in SEQ ID NO:15; and
   L-CDR1 has the amino acid sequence shown in SEQ ID NO:4, L-CDR2 has the amino acid sequence shown in SEQ ID NO:16 and L-CDR3 has the amino acid sequence shown in SEQ ID NO:17; or
(4) H-CDR1 has the amino acid sequence shown in SEQ ID NO:18, H-CDR2 has the amino acid sequence shown in SEQ ID NO:19 and H-CDR3 has the amino acid sequence shown in SEQ ID NO:20; and
   L-CDR1 has the amino acid sequence shown in SEQ ID NO:4, L-CDR2 has the amino acid sequence shown in SEQ ID NO:16 and L-CDR3 has the amino acid sequence shown in SEQ ID NO:21; or
(5) H-CDR1 has the amino acid sequence shown in SEQ ID NO:22, H-CDR2 has the amino acid sequence shown in SEQ ID NO:23 and H-CDR3 has the amino acid sequence shown in SEQ ID NO:24; and
   L-CDR1 has the amino acid sequence shown in SEQ ID NO:25, L-CDR2 has the amino acid sequence shown in SEQ ID NO:26 and L-CDR3 has the amino acid sequence shown in SEQ ID NO:27; or
(6) H-CDR1 has the amino acid sequence shown in SEQ ID NO:28, H-CDR2 has the amino acid sequence shown in SEQ ID NO:29 and H-CDR3 has the amino acid sequence shown in SEQ ID NO:30; and
   L-CDR1 has the amino acid sequence shown in SEQ ID NO:31, L-CDR2 has the amino acid sequence shown in SEQ ID NO:32 and L-CDR3 has the amino acid sequence shown in SEQ ID NO:33; or
(7) H-CDR1 has the amino acid sequence shown in SEQ ID NO:34, H-CDR2 has the amino acid sequence shown in SEQ ID NO:35 and H-CDR3 has the amino acid sequence shown in SEQ ID NO:36; and
   L-CDR1 has the amino acid sequence shown in SEQ ID NO:37, L-CDR2 has the amino acid sequence shown in SEQ ID NO:38 and L-CDR3 has the amino acid sequence shown in SEQ ID NO:39; or
(8) H-CDR1 has the amino acid sequence shown in SEQ ID NO:40, H-CDR2 has the amino acid sequence shown in SEQ ID NO:41 and H-CDR3 has the amino acid sequence shown in SEQ ID NO: 42; and
   L-CDR1 has the amino acid sequence shown in SEQ ID NO:43, L-CDR2 has the amino acid sequence shown in SEQ ID NO:44 and L-CDR3 has the amino acid sequence shown in SEQ ID NO:45; or
(9) H-CDR1 has the amino acid sequence shown in SEQ ID NO:46, H-CDR2 has the amino acid sequence shown in SEQ ID NO:47 and H-CDR3 has the amino acid sequence shown in SEQ ID NO: 48; and
   L-CDR1 has the amino acid sequence shown in SEQ ID NO:37, L-CDR2 has the amino acid sequence shown in SEQ ID NO:38 and L-CDR3 has the amino acid sequence shown in SEQ ID NO:49; or
(10) H-CDR1 has the amino acid sequence shown in SEQ ID NO:50, H-CDR2 has the amino acid sequence shown in SEQ ID NO:51 and H-CDR3 has the amino acid sequence shown in SEQ ID NO: 52; and
   L-CDR1 has the amino acid sequence shown in SEQ ID NO:53, L-CDR2 has the amino acid sequence shown in SEQ ID NO:54 and L-CDR3 has the amino acid sequence shown in SEQ ID NO: 45.
(11) H-CDR1 has the amino acid sequence shown in SEQ ID NO:127, H-CDR2 has the amino acid sequence shown in SEQ ID NO:29 and H-CDR3 has the amino acid sequence shown in SEQ ID NO: 128; and
   L-CDR1 has the amino acid sequence shown in SEQ ID NO:129, L-CDR2 has the amino acid sequence shown in SEQ ID NO:130 and L-CDR3 has the amino acid sequence shown in SEQ ID NO: 131.
(12) H-CDR1 has the amino acid sequence shown in SEQ ID NO:18, H-CDR2 has the amino acid sequence shown in SEQ ID NO:29 and H-CDR3 has the amino acid sequence shown in SEQ ID NO: 132; and
   L-CDR1 has the amino acid sequence shown in SEQ ID NO:37, L-CDR2 has the amino acid sequence shown in SEQ ID NO:133 and L-CDR3 has the amino acid sequence shown in SEQ ID NO: 134.
(13) H-CDR1 has the amino acid sequence shown in SEQ ID NO:18, H-CDR2 has the amino acid sequence shown in SEQ ID NO:29 and H-CDR3 has the amino acid sequence shown in SEQ ID NO: 128; and
   L-CDR1 has the amino acid sequence shown in SEQ ID NO:135, L-CDR2 has the amino acid sequence shown in SEQ ID NO:136 and L-CDR3 has the amino acid sequence shown in SEQ ID NO: 137.
(14) H-CDR1 has the amino acid sequence shown in SEQ ID NO:138, H-CDR2 has the amino acid sequence shown in SEQ ID NO:139 and H-CDR3 has the amino acid sequence shown in SEQ ID NO: 140; and
   L-CDR1 has the amino acid sequence shown in SEQ ID NO:141, L-CDR2 has the amino acid sequence shown in SEQ ID NO:142 and L-CDR3 has the amino acid sequence shown in SEQ ID NO: 143.

In some embodiments, the antibody or antigen-binding fragment comprises a heavy chain variable region framework sequence and a light chain variable region framework sequence, wherein the framework sequence is derived from a murine antibody, a human antibody, a primate-derived antibody, or mutants thereof.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:55-64 or SEQ ID NO:144-147.

In some embodiments, the light chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:65-76 or SEQ ID NO:148-151.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:55; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:65.

In some embodiments, the heavy chain variable region of the antibody or antigen-binding fragment comprises the amino acid sequence shown in SEQ ID NO:55; and the light chain variable region comprises the amino acid sequence shown in SEQ ID NO:65.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:56; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:66.

In some embodiments, the heavy chain variable region of the antibody or antigen-binding fragment comprises an amino acid sequence shown in SEQ ID NO:56; and the light chain variable region comprises the amino acid sequence shown in SEQ ID NO:66.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:57; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:67.

In some embodiments, the heavy chain variable region of the antibody or antigen-binding fragment comprises an amino acid sequence shown in SEQ ID NO:57; and the light chain variable region comprises the amino acid sequence shown in SEQ ID NO:67.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:58; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:68.

In some embodiments, the heavy chain variable region of the antibody or antigen-binding fragment comprises an amino acid sequence shown in SEQ ID NO:58; and the light chain variable region comprises the amino acid sequence shown in SEQ ID NO:68.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:59; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:69.

In some embodiments, the heavy chain variable region of the antibody or antigen-binding fragment comprises an amino acid sequence shown in SEQ ID NO:59; and the light chain variable region comprises the amino acid sequence shown in SEQ ID NO:69.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:60; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:70.

In some embodiments, the heavy chain variable region of the antibody or antigen-binding fragment comprises an amino acid sequence shown in SEQ ID NO:60; and the light chain variable region comprises the amino acid sequence shown in SEQ ID NO:70.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:61; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:71.

In some embodiments, the heavy chain variable region of the antibody or antigen-binding fragment comprises an amino acid sequence shown in SEQ ID NO:61; and the light chain variable region comprises the amino acid sequence shown in SEQ ID NO:71.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:62; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:72 or 73.

In some embodiments, the heavy chain variable region of the antibody or antigen-binding fragment comprises an amino acid sequence shown in SEQ ID NO:62; and the light chain variable region comprises the amino acid sequence shown in SEQ ID NO:72 or 73.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:63; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:74.

In some embodiments, the heavy chain variable region of the antibody or antigen-binding fragment comprises the amino acid sequence shown in SEQ ID NO:63; and the light chain variable region comprises the amino acid sequence shown in SEQ ID NO:74.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:64; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:75 or 76.

In some embodiments, the heavy chain variable region of the antibody or antigen-binding fragment comprises the amino acid sequence shown in SEQ ID NO:64; and the light chain variable region comprises the amino acid sequence shown in SEQ ID NO:75 or 76.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:144; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:148.

In some embodiments, the heavy chain variable region of the antibody or antigen-binding fragment comprises the amino acid sequence shown in SEQ ID NO:144; and the light chain variable region comprises the amino acid sequence shown in SEQ ID NO:148.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:145; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:149.

In some embodiments, the heavy chain variable region of the antibody or antigen-binding fragment comprises the amino acid sequence shown in SEQ ID NO:145; and the light chain variable region comprises the amino acid sequence shown in SEQ ID NO:149.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:146; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:150.

In some embodiments, the heavy chain variable region of the antibody or antigen-binding fragment comprises the amino acid sequence shown in SEQ ID NO:146; and the light chain variable region comprises the amino acid sequence shown in SEQ ID NO:150.

In some embodiments, the heavy chain variable region of the antibody or antigen-binding fragment comprises an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:147; and the light chain variable region comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO:151.

In some embodiments, the heavy chain variable region of the antibody or antigen-binding fragment comprises the amino acid sequence shown in SEQ ID NO:147; and the light chain variable region comprises the amino acid sequence shown in SEQ ID NO:151.

EVHLVESGGGVVQPGRSLRLSCAASGFTFSNYAMHWVRQAPGKGLEWVAVISYDGSK
KYYADSVKGRFTISRDDSKNTLYLQMNSLRAEDTAVYYCAKPAGSTSGFDYWGQGTLVTV
SS (SEQ ID NO: 55).

QSALTQPASVSGSPGQSITISCTGTSSDVGGYNYVSWYQQHPGKAPKLMIYDVSNRPSG
VSNRFSGSKSGNTASLTISGLQAEDEADYYCSSYTSSSTLVVFGGGTKLTVL (SEQ ID NO: 65).

QVQLVQSGGGVVRPGGSLRLSCAASGFTFDDYGMSWVRQAPGKGLEWVSGINWNGG
STGYADSVKGRFTISRDNSKNTLYLQMNSLRAEDTAVYYCATSSVKHYWGQGTLVTVSS
(SEQ ID NO: 56).

QPVLTQPPSASATPGQRVTISCSGSSSNIGTNTVNWYQQVPGTAPKVLIYRNDLRPSGVP
DRFSGSKSGTSASLAISGLQSDDEADYYCAAWDDSLNGVVFGGGTKVTVL (SEQ ID NO:
66).

QVQLVQSGPEMKKPGASVKVACKTSGYIFINYGITWVRQAPGQGLEWMGWISPYNGH
TIYAQKVQGRGTMTTETPTSTAYLDLTNLTPDDTAIYYCATGLNSRLQYWGQGALVTVSS

(SEQ ID NO: 57).

QPVLTQPASVSGSPGQSITISCTGTSSDVGGYNYVSWYQQHPGKAPKLMIYDVSKRPSG
VSNRFSGSKSGNTASLTISGLQAEDEADYYCSSYTSSSTDVFGTGTKVTVL (SEQ ID NO: 67).

EVQLVQSGAEVKKPGASMKVSCKASGYTFTGYYMHWVRQAPGQGLEWMGWINPVS
GGTNYAQKFQGRVTMTRDTSIGTAYMELTGLRSDDTAVYYCAADPFDIWGQGTMVTVSS
(SEQ ID NO: 58).

QSALTQPASVSGSPGQSITISCTGTSSDVGGYNYVSWYQQHPGKAPKLMIYDVSKRPSG
VSNRFSGSKSGNTASLTISGLQAEDEADYYCSSYTSRSTPGYVFGTGTKLTVL (SEQ ID NO:
68).

QVQLVQSGAEVKKPGASVKVSCKASGYTFTNYGFSWVRQAPGQGLEWMGWISSYNG
NTKYAQKFRGRVTMTTDTSTSTGYMELRSLRSDDTAVYYCARGGDWPADYWGQGTLVTV
SS (SEQ ID NO: 59).

QAVVTQEPSFSVSPGGTVTLTCGLSSGSVSNNYYPSWYQQIPGQAPRTLIYSTNTRSSGV
PDRFSGSILGNKAALTITGAQADDEADYYCVVYMGSGIWVFGGGTKLTVL (SEQ ID NO: 69).

QVQLVQSGAEVKKPGASVKVSCKASGYTFTSYGISWVRQAPGQGLEWMGWINPNSGG
TNYAQKFQGRVTMTRDTSISTAYMELSRLRSDDTAVYYCARAGDLRDYYYYGMDVWGQG
TTVTVSS (SEQ ID NO: 60).

QSVLTQPPSVSGAPGQRVTISCTGSSSNIGAGYDVHWYQQLPGTAPKLLIYGNSNRPSGV
PDRFSGSKSGTSASLAITGLQAEDEADYYCQSYDSSLSGWVFGGGTQLTAL (SEQ ID NO:
70).

QVQLVQSGAEVKKPGASVKVSCKASGYTFTNHFIHWVRQAPGQGLEWMGWISANSG
NTDYAQKFQGRVTMTTDTSTSTAYMELRSLRYDDTAVYYCARDLATVDGFDIWGQGTMVT
VSS (SEQ ID NO: 61).

DVVMTQSPLSLPVTPGEPASISCRSSQSLLHSNGYNYLDWYLQKPGQSPQLLIYLGSNR
ASGVPDRFSGSGSGTDFTLKISRVEAEDVGVYYCMQALQTPLTFGPGTKVDIK (SEQ ID NO:
71).

EVQLVQSGSEVKKPGSSVKVSCKAPGGTFSSYSVSWVRQAPGQGLEWMGGIIPTFGRT
NYAQKFQGRVTISAHESTGTAYMELSSLRSEDTAVYYCASVRKPYYGMDVWGQGTMVTVS
S (SEQ ID NO: 62).

DIQMTQSPSSLSASVGDRVTITCRASQSIGRFLNWYQQKPGTAPKLLISGASRLQGGIPS
RFSGSGSGTDFTLTISSLQPEDFATYYCQQSYSTPPTFGQGTKVEIK (SEQ ID NO: 72).

AIWMTQSPSSLSASVGDRVTITCRASQSIGRFLNWYQQKPGTAPKLLISGASRLQGGIPS
RFSGSGSGTDFTLTISSLQPEDFATYYCQQSYSTPPTFGQGTKVEIK (SEQ ID NO: 73).

QVQLVQSGSELKKPGASVKISCKASGYTLTSYGINWVRQAPGKGLELMGWINTKTGNP
TYVQGFTGQVVFSFDTSVNTAYLQINNLKSDDTAVYFCARDRNPYGMDVWGQGTTVTVSS
(SEQ ID NO: 63).

EIVLTQSPLSLPVTPGEPASISCRSSQSLLHSNGYNYLDWYLQKPGQSPQLLIYLGSNRAS

GVPDRFSGSGSGTDFTLKISRVEAEDVGVYYCMQPLQALYTFGQGTKVEIK (SEQ ID NO:
74).

QVQLVQSGAEVKKPGSSVKVSCKASGGTFSSYAISWVRQAPGQGLEWMGGIIPIFGTA
NYAQKFQGRVTITADESTSTAYMELSSLRSEDTAVYYCARTGGDYYYYGMDVWGQGTTVT
VSS (SEQ ID NO: 64).

DIQMTQSPSSLSASVGDRVTITCRASQTISTYLNWYQQKPGKAPKLLIYGASSLQTGVP
SRFSGSGSGTDFTLTISSLQPEDFATYYCQQSYSTPPTFGQGTKLEIK (SEQ ID NO: 75).

AIWMTQSPSSLSASVGDRVTITCRASQTISTYLNWYQQKPGKAPKLLIYGASSLQTGVP
SRFSGSGSGTDFTLTISSLQPEDFATYYCQQSYSTPPTFGQGTKVDIK (SEQ ID NO: 76).

EVQLVESGAEVKKPGASVKVSCKASGYTFTDYFLHWVRQAPGQGLEWMGWINPNSG
GTNYAQKFQGWVTMTRDTSISTAYLELSSLTSDDTAVYYCAVDPFDYWGQGTLVTVSS
(SEQ ID NO: 144).

QVQLVQSGAEVKKPGASVKVSCKASGYTFTGYYMHWVRQAPGQGLEWMGWINPNS
GGTNYAQKFQGRVTMTRDTSISTAYMELSRLRSDDTAVYYCARVGGSYYGGFDYWGQGTL
VTVSS (SEQ ID NO: 145).

QVQLVQSGAEVKKPGASVKVSCKASGYTFTGYYMHWVRQAPGQGLEWMGWINPNS
GGTNYAQKFQGRVTMTRDTSISTAYMELSRLRSDDTAVYYCASDPFDYWGQGTLVTVSS
(SEQ ID NO: 146).

QVQLVQSGAEVKKPGESLKISCKGSGYSFTNYWIGWVRQMPGKGLEWMGIIYPGDSD
TRYSPSFQGQVTISADKSISTAYLQWSSLKASDTAMYYCARPQGAFDIWGQGTMVTVSS
(SEQ ID NO: 147).

DVVMTQSPLSSPVTLGQPASISCRSSQSLVHSDGNTYLSWLHQRPGQPPRLLIHKISNRF
SGVPDRFSGSGAGTDFTLKISRVEAEDVGVFYCMQATQFPITFGQGTRLEIK (SEQ ID NO:
148).

EIVLTQSPLSLPVTPGEPASISCRSSQSLLHSNGYNYLDWYLQKPGQSPQLLMYLSSNR
ASGVPDRFSGSGSGRDFTLKISRVEAEDVGLYYCMQALQTPPTFGQGTRLEIK (SEQ ID NO:
149).

DVVMTQSPLSSPVTLGQPASISCRSSQSLVYSDGNTYLSWLQQRPGQPPRLLIYKVSNR
FSGVPDRFSGSGAGTDFTLTISRVEAEDVGVYYCMQATQYPLAFGGGTKLEIK (SEQ ID NO:
150).

EIVMTQTPLSLPVTLGQPASISCRSSQSLVYSDGNTYLNWFQQRPGQSPRRLIYKVSNR
DSGVPDRFSGSGSGTDFTLKISRVEAEDVGVYYCMQGTHWPLTFGQGTKVEIK (SEQ ID
NO: 151).

In some embodiments, the heavy chain variable region and the light chain variable region of the extracellular antigen-binding domain are connected via a linker.

The "Linker" is used to connect the heavy chain variable region and the light chain variable region of the antibody, or a peptide Linker modified by researchers. An example of the amino acid sequence of the Linker is: (GnS)m, wherein n is a natural number from 3 to 5, preferably 4, and m is a natural number from 1 to 5, preferably 3.
GGGS (SEQ ID NO: 77).
GGGGS (SEQ ID NO: 78).
GGGGSGGGGS (SEQ ID NO: 79).
GGGGSGGGGSGGGGS (SEQ ID NO: 80).

In some embodiments, the extracellular antigen-binding domain is single-chain variable fragment (scFv).

The extracellular antigen-binding domain comprises a single-chain variable fragment (scFv) comprising an amino acid sequence having at least 85%, at least 90%, at least 91%, at least 92%, at least 93%, at least 94%, at least 95%, at least 96%, at least 97%, at least 98%, or at least 99% sequence identity to any one of SEQ ID NO:81-90 or SEQ ID NO:152-155.

EVHLVESGGGVVQPGRSLRLSCAASGFTFSNYAMHWVRQAPGKGLEWVAVISYDGSK
KYYADSVKGRFTISRDDSKNTLYLQMNSLRAEDTAVYYCAKPAGSTSGFDYWGQGTLVTV
SSGGGGSGGGGSGGGGSQSALTQPASVSGSPGQSITISCTGTSSDVGGYNYVSWYQQHPGK
APKLMIYDVSNRPSGVSNRFSGSKSGNTASLTISGLQAEDEADYYCSSYTSSSTLVVFGGGT
KLTVL (SEQ ID NO: 81).

QVQLVQSGGGVVRPGGSLRLSCAASGFTFDDYGMSWVRQAPGKGLEWVSGINWNG
GSTGYADSVKGRFTISRDNSKNTLYLQMNSLRAEDTAVYYCATSSVKHYWGQGTLVTVSS
GGGGSGGGGSGGGGSQPVLTQPPSASATPGQRVTISCSGSSSNIGTNTVNWYQQVPGTAPK
VLIYRNDLRPSGVPDRFSGSKSGTSASLAISGLQSDDEADYYCAAWDDSLNGVVFGGGTKV
TVL (SEQ ID NO: 82).

QVQLVQSGPEMKKPGASVKVACKTSGYIFINYGITWVRQAPGQGLEWMGWISPYNGH
TIYAQKVQGRGTMTTETPTSTAYLDLTNLTPDDTAIYYCATGLNSRLQYWGQGALVTVSSG
GGGSGGGGSGGGGSQPVLTQPASVSGSPGQSITISCTGTSSDVGGYNYVSWYQQHPGKAPK
LMIYDVSKRPSGVSNRFSGSKSGNTASLTISGLQAEDEADYYCSSYTSSSTDVFGTGTKVTV
L (SEQ ID NO: 83).

EVQLVQSGAEVKKPGASMKVSCKASGYTFTGYYMHWVRQAPGQGLEWMGWINPVS
GGTNYAQKFQGRVTMTRDTSIGTAYMELTGLRSDDTAVYYCAADPFDIWGQGTMVTVSSG
GGGSGGGGSGGGGSQSALTQPASVSGSPGQSITISCTGTSSDVGGYNYVSWYQQHPGKAPK
LMIYDVSKRPSGVSNRFSGSKSGNTASLTISGLQAEDEADYYCSSYTSRSTPGYVFGTGTKL
TVL (SEQ ID NO: 84).

QVQLVQSGAEVKKPGASVKVSCKASGYTFTNYGFSWVRQAPGQGLEWMGWISSYN
GNTKYAQKFRGRVTMTTDTSTSTGYMELRSLRSDDTAVYYCARGGDWPADYWGQGTLVT
VSSGGGGSGGGGSGGGGSQAVVTQEPSFSVSPGGTVTLTCGLSSGSVSNNYYPSWYQQIPG
QAPRTLIYSTNTRSSGVPDRFSGSILGNKAALTITGAQADDEADYYCVVYMGSGIWVFGGG
TKLTVL (SEQ ID NO: 85).

QVQLVQSGAEVKKPGASVKVSCKASGYTFTSYGISWVRQAPGQGLEWMGWINPNSG
GTNYAQKFQGRVTMTRDTSISTAYMELSRLRSDDTAVYYCARAGDLRDYYYYGMDVWGQ
GTTVTVSSGGGGSGGGGSGGGGSQSVLTQPPSVSGAPGQRVTISCTGSSSNIGAGYDVHWY
QQLPGTAPKLLIYGNSNRPSGVPDRFSGSKSGTSASLAITGLQAEDEADYYCQSYDSSLSGW
VFGGGTQLTAL (SEQ ID NO: 86).

QVQLVQSGAEVKKPGASVKVSCKASGYTFTNHFIHWVRQAPGQGLEWMGWISANSG
NTDYAQKFQGRVTMTTDTSTSTAYMELRSLRYDDTAVYYCARDLATVDGFDIWGQGTMVT
VSSGGGGSGGGGSGGGGSDVVMTQSPLSLPVTPGEPASISCRSSQSLLHSNGYNYLDWYLQ
KPGQSPQLLIYLGSNRASGVPDRFSGSGSGTDFTLKISRVEAEDVGVYYCMQALQTPLTFGP
GTKVDIK (SEQ ID NO: 87).

EVQLVQSGSEVKKPGSSVKVSCKAPGGTFSSYSVSWVRQAPGQGLEWMGGIIPTFGRT
NYAQKFQGRVTISAHESTGTAYMELSSLRSEDTAVYYCASVRKPYYGMDVWGQGTMVTVS
SGGGGSGGGGSGGGGSAIWMTQSPSSLASVGDRVTITCRASQSIGRFLNWYQQKPGTAPK
LLISGASRLQGGIPSRFSGSGSGTDFTLTISSLQPEDFATYYCQQSYSTPPTFGQGTKVEIK
(SEQ ID NO: 88).

QVQLVQSGSELKKPGASVKISCKASGYTLTSYGINWVRQAPGKGLELMGWINTKTGN
PTYVQGFTGQVVFSFDTSVNTAYLQINNLKSDDTAVYFCARDRNPYGMDVWGQGTTVTVS
SGGGGSGGGGSGGGGSEIVLTQSPLSLPVTPGEPASISCRSSQSLLHSNGYNYLDWYLQKPG
QSPQLLIYLGSNRASGVPDRFSGSGSGTDFTLKISRVEAEDVGVYYCMQPLQALYTFGQGT
KVEIK (SEQ ID NO: 89).

QVQLVQSGAEVKKPGSSVKVSCKASGGTFSSYAISWVRQAPGQGLEWMGGIIPIFGTA
NYAQKFQGRVTITADESTSTAYMELSSLRSEDTAVYYCARTGGDYYYYGMDVWGQGTTVT
VSSGGGGSGGGGSGGGGSAIWMTQSPSSLSASVGDRVTITCRASQTISTYLNWYQQKPGKA
PKLLIYGASSLQTGVPSRFSGSGSGTDFTLTISSLQPEDFATYYCQQSYSTPPTFGQGTKVDIK
(SEQ ID NO: 90).

EVQLVESGAEVKKPGASVKVSCKASGYTFTDYFLHWVRQAPGQGLEWMGWINPNSG
GTNYAQKFQGWVTMTRDTSISTAYLELSSLTSDDTAVYYCAVDPFDYWGQGTLVTVSSGGG
GSGGGGSGGGGSDVVMTQSPLSSPVTLGQPASISCRSSQSLVHSDGNTYLSWLHQRPGQPP
RLLIHKISNRFSGVPDRFSGSGAGTDFTLKISRVEAEDVGVFYCMQATQFPITFGQGTRLEIK
(SEQ ID NO: 152).

QVQLVQSGAEVKKPGASVKVSCKASGYTFTGYYMHWVRQAPGQGLEWMGWINPNS
GGTNYAQKFQGRVTMTRDTSISTAYMELSRLRSDDTAVYYCARVGGSYYGGFDYWGQGTL
VTVSSGGGGSGGGGSGGGGSEIVLTQSPLSLPVTPGEPASISCRSSQSLLHSNGYNYLDWYL
QKPGQSPQLLMYLSSNRASGVPDRFSGSGSGRDFTLKISRVEAEDVGLYYCMQALQTPPTF
GQGTRLEIK (SEQ ID NO: 153).

QVQLVQSGAEVKKPGASVKVSCKASGYTFTGYYMHWVRQAPGQGLEWMGWINPNS
GGTNYAQKFQGRVTMTRDTSISTAYMELSRLRSDDTAVYYCASDPFDYWGQGTLVTVSSG

GGGSGGGGSGGGGSDVVMTQSPLSSPVTLGQPASISCRSSQSLVYSDGNTYLSWLQQRPGQ
PPRLLIYKVSNRFSGVPDRFSGSGAGTDFTLTISRVEAEDVGVYYCMQATQYPLAFGGGTKL
EIK (SEQ ID NO: 154).

QVQLVQSGAEVKKPGESLKISCKGSGYSFTNYWIGWVRQMPGKGLEWMGIIYPGDSD
TRYSPSFQGQVTISADKSISTAYLQWSSLKASDTAMYYCARPQGAFDIWGQGTMVTVSSGG
GGSGGGGSGGGGSEIVMTQTPLSLPVTLGQPASISCRSSQSLVYSDGNTYLNWFQQRPGQSP
RRLIYKVSNRDSGVPDRFSGSGSGTDFTLKISRVEAEDVGVYYCMQGTHWPLTFGQGTKVE
IK (SEQ ID NO: 155).

In some embodiments, the transmembrane domain comprises a transmembrane domain derived from a protein selected from the group consisting of: the α, β, or ζ chain of a T cell receptor, CD28, CD3ζ, CD45, CD4, CD5, CD8α, CD9, CD16, CD22, CD33, CD37, CD64, CD80, CD86, CD134, 4-1BB, CD154, or combinations thereof.

In some embodiments, the transmembrane domain comprises a transmembrane domain derived from the CD8α protein.

In some embodiments, the transmembrane domain comprises the amino acid sequence shown in SEQ ID NO:92.
IWAPLAGTCGVLLLSLVITLYC (SEQ ID NO: 92).

In some embodiments, the chimeric antigen receptor further comprises a hinge region.

The hinge region generally refers to a linking region between the antigen-binding region and the immune cell Fc receptor (FcR) binding region.

In some embodiments, the hinge region connects the extracellular antigen-binding domain and the transmembrane domain.

In some embodiments, the hinge region comprises the amino acid sequence shown in SEQ ID NO:93.
TTTPAPRPPTPAPTIASQPLSLRPEACRPAAGGAVHTRGLDFACDIY (SEQ ID NO: 93).

In some embodiments, the intracellular domain of the chimeric antigen receptor comprises a signaling domain derived from CD3ζ.

In some embodiments, the intracellular domain of the chimeric antigen receptor comprises the amino acid sequence shown in SEQ ID NO:94.

RVKFSRSADAPAYQQGQNQLYNELNLGRREEYDVLDKRRGRDPEMGGKPQRRKNPQ
EGLYNELQKDKMAEAYSEIGMKGERRRGKGHDGLYQGLSTATKDTYDALHMQALPPR
(SEQ ID NO: 94).

In some embodiments, the intracellular domain further comprises at least one costimulatory domain.

In some embodiments, the costimulatory domain comprises a costimulatory domain derived from a protein selected from the group consisting of: CD28, 4-1BB, OX-40, ICOS, or combinations thereof.

In some embodiments, the costimulatory domain comprises a domain derived from 4-1BB.

In some embodiments, the costimulatory domain comprises the amino acid sequence shown in SEQ ID NO:95.
KRGRKKLLYIFKQPFMRPVQTTQEEDGCSCRFPEEEEGGCEL (SEQ ID NO: 95).

In some embodiments, the costimulatory domain comprises a domain derived from ICOS.

In some embodiments, the costimulatory domain comprises the amino acid sequence shown in SEQ ID NO:96.
CWLTKKKYSSSVHDPNGEYMFMRAVNTAKKSRLTDVTL (SEQ ID NO: 96).

In some embodiments, the costimulatory domain comprises a domain derived from a combination of 4-1BB and ICOS.

In some embodiments, the costimulatory domain comprises the amino acid sequence shown in SEQ ID NO:97.

CWLTKKKYSSSVHDPNGEYMFMRAVNTAKKSRLTDVTLKRGRKKLLYIFKQPFMRPV
QTTQEEDGCSCRFPEEEEGGCEL (SEQ ID NO: 97).

In some embodiments, the intracellular domain comprises the amino acid sequence shown in SEQ ID NO:98.

CWLTKKKYSSSVHDPNGEYMFMRAVNTAKKSRLTDVTLKRGRKKLLYIFKQPFMRPV
QTTQEEDGCSCRFPEEEEGGCELRVKFSRSADAPAYQQGQNQLYNELNLGRREEYDVLDKR
RGRDPEMGGKPQRRKNPQEGLYNELQKDKMAEAYSEIGMKGERRRGKGHDGLYQGLSTA
TKDTYDALHMQALPPR (SEQ ID NO: 98).

In some embodiments, the chimeric antigen receptor comprises an amino acid sequence shown in any one of SEQ ID NO:99-108 or SEQ ID NO:156-159.

Secifically, the chimeric antigen receptor D9-CAR comprises the amino acid sequence shown in SEQ ID NO:99.

EVHLVESGGGVVQPGRSLRLSCAASGFTFSNYAMHWVRQAPGKGLEWVAVISYDGSK
KYYADSVKGRFTISRDDSKNTLYLQMNSLRAEDTAVYYCAKPAGSTSGFDYWGQGTLVTV
SSGGGGSGGGGSGGGGSQSALTQPASVSGSPGQSITISCTGTSSDVGGYNYVSWYQQHPGK
APKLMIYDVSNRPSGVSNRFSGSKSGNTASLTISGLQAEDEADYYCSSYTSSSTLVVFGGGT
KLTVLTTTPAPRPPTPAPTIASQPLSLRPEACRPAAGGAVHTRGLDFACDIYIWAPLAGTCGV
LLLSLVITLYCCWLTKKKYSSSVHDPNGEYMFMRAVNTAKKSRLTDVTLKRGRKKLLYIFK
QPFMRPVQTTQEEDGCSCRFPEEEEGGCELRVKFSRSADAPAYQQGQNQLYNELNLGRREE
YDVLDKRRGRDPEMGGKPQRRKNPQEGLYNELQKDKMAEAYSEIGMKGERRRGKGHDG
LYQGLSTATKDTYDALHMQALPPR (SEQ ID NO: 99).

Secifically, the chimeric antigen receptor D10-CAR comprises the amino acid sequence shown in SEQ ID NO:100.

QVQLVQSGGGVVRPGGSLRLSCAASGFTFDDYGMSWVRQAPGKGLEWVSGINWNG
GSTGYADSVKGRFTISRDNSKNTLYLQMNSLRAEDTAVYYCATSSVKHYWGQGTLVTVSS

GGGGSGGGGSGGGGSQPVLTQPPSASATPGQRVTISCSGSSSNIGTNTVNWYQQVPGTAPK
VLIYRNDLRPSGVPDRFSGSKSGTSASLAISGLQSDDEADYYCAAWDDSLNGVVFGGGTKV
TVLTTTPAPRPPTPAPTIASQPLSLRPEACRPAAGGAVHTRGLDFACDIYIWAPLAGTCGVLL
LSLVITLYCCWLTKKKYSSSVHDPNGEYMFMRAVNTAKKSRLTDVTLKRGRKKLLYIFKQP
FMRPVQTTQEEDGCSCRFPEEEEGGCELRVKFSRSADAPAYQQGQNQLYNELNLGRREEYD
VLDKRRGRDPEMGGKPQRRKNPQEGLYNELQKDKMAEAYSEIGMKGERRRGKGHDGLY
QGLSTATKDTYDALHMQALPPR (SEQ ID NO: 100).

Secifically, the chimeric antigen receptor D28-CAR comprises the amino acid sequence shown in SEQ ID NO:101.

QVQLVQSGPEMKKPGASVKVACKTSGYIFINYGITWVRQAPGQGLEWMGWISPYNGH
TIYAQKVQGRGTMTTETPTSTAYLDLTNLTPDDTAIYYCATGLNSRLQYWGQGALVTVSSG
GGGSGGGGSGGGGSQPVLTQPASVSGSPGQSITISCTGTSSDVGGYNYVSWYQQHPGKAPK
LMIYDVSKRPSGVSNRFSGSKSGNTASLTISGLQAEDEADYYCSSYTSSSTDVFGTGTKVTV
LTTTPAPRPPTPAPTIASQPLSLRPEACRPAAGGAVHTRGLDFACDIYIWAPLAGTCGVLLLSL
VITLYCCWLTKKKYSSSVHDPNGEYMFMRAVNTAKKSRLTDVTLKRGRKKLLYIFKQPFM
RPVQTTQEEDGCSCRFPEEEEGGCELRVKFSRSADAPAYQQGQNQLYNELNLGRREEYDVL
DKRRGRDPEMGGKPQRRKNPQEGLYNELQKDKMAEAYSEIGMKGERRRGKGHDGLYQG
LSTATKDTYDALHMQALPPR (SEQ ID NO: 101).

Secifically, the chimeric antigen receptor D30-CAR comprises the amino acid sequence shown in SEQ ID NO:102.

EVQLVQSGAEVKKPGASMKVSCKASGYTFTGYYMHWVRQAPGQGLEWMGWINPVS
GGTNYAQKFQGRVTMTRDTSIGTAYMELTGLRSDDTAVYYCAADPFDIWGQGTMVTVSSG
GGGSGGGGSGGGGSQSALTQPASVSGSPGQSITISCTGTSSDVGGYNYVSWYQQHPGKAPK
LMIYDVSKRPSGVSNRFSGSKSGNTASLTISGLQAEDEADYYCSSYTSRSTPGYVFGTGTKL
TVLTTTPAPRPPTPAPTIASQPLSLRPEACRPAAGGAVHTRGLDFACDIYIWAPLAGTCGVLL
LSLVITLYCCWLTKKKYSSSVHDPNGEYMFMRAVNTAKKSRLTDVTLKRGRKKLLYIFKQP
FMRPVQTTQEEDGCSCRFPEEEEGGCELRVKFSRSADAPAYQQGQNQLYNELNLGRREEYD
VLDKRRGRDPEMGGKPQRRKNPQEGLYNELQKDKMAEAYSEIGMKGERRRGKGHDGLY
QGLSTATKDTYDALHMQALPPR (SEQ ID NO: 102).

Secifically, the chimeric antigen receptor D32-CAR comprises the amino acid sequence shown in SEQ ID NO:103.

QVQLVQSGAEVKKPGASVKVSCKASGYTFTNYGFSWVRQAPGQGLEWMGWISSYN
GNTKYAQKFRGRVTMTTDTSTSTGYMELRSLRSDDTAVYYCARGGDWPADYWGQGTLVT
VSSGGGGSGGGGSGGGGSQAVVTQEPSFSVSPGGTVTLTCGLSSGSVSNNYYPSWYQQIPG
QAPRTLIYSTNTRSSGVPDRFSGSILGNKAALTITGAQADDEADYYCVVYMGSGIWVFGGG
TKLTVLTTTPAPRPPTPAPTIASQPLSLRPEACRPAAGGAVHTRGLDFACDIYIWAPLAGTCG
VLLLSLVITLYCCWLTKKKYSSSVHDPNGEYMFMRAVNTAKKSRLTDVTLKRGRKKLLYIF

KQPFMRPVQTTQEEDGCSCRFPEEEEGGCELRVKFSRSADAPAYQQGQNQLYNELNLGRRE
EYDVLDKRRGRDPEMGGKPQRRKNPQEGLYNELQKDKMAEAYSEIGMKGERRRGKGHD
GLYQGLSTATKDTYDALHMQALPPR (SEQ ID NO: 103).

Secifically, the chimeric antigen receptor D46-CAR comprises the amino acid sequence shown in SEQ ID NO:104.

QVQLVQSGAEVKKPGASVKVSCKASGYTFTSYGISWVRQAPGQGLEWMGWINPNSGGTN
YAQKFQGRVTMTRDTSISTAYMELSRLRSDDTAVYYCARAGDLRDYYYYGMDVWGQGTT
VTVSSGGGGSGGGGSGGGGSQSVLTQPPSVSGAPGQRVTISCTGSSSNIGAGYDVHWYQQL
PGTAPKLLIYGNSNRPSGVPDRFSGSKSGTSASLAITGLQAEDEADYYCQSYDSSLSGWVFG
GGTQLTALTTTPAPRPPTPAPTIASQPLSLRPEACRPAAGGAVHTRGLDFACDIYIWAPLAGTC
GVLLLSLVITLYCCWLTKKKYSSSVHDPNGEYMFMRAVNTAKKSRLTDVTLKRGRKKLLYI
FKQPFMRPVQTTQEEDGCSCRFPEEEEGGCELRVKFSRSADAPAYQQGQNQLYNELNLGRR
EEYDVLDKRRGRDPEMGGKPQRRKNPQEGLYNELQKDKMAEAYSEIGMKGERRRGKGH
DGLYQGLSTATKDTYDALHMQALPPR (SEQ ID NO: 104).

Secifically, the chimeric antigen receptor D59-CAR comprises the amino acid sequence shown in SEQ ID NO:105.

QVQLVQSGAEVKKPGASVKVSCKASGYTFTNHFIHWVRQAPGQGLEWMGWISANSG
NTDYAQKFQGRVTMTTDTSTSTAYMELRSLRYDDTAVYYCARDLATVDGFDIWGQGTMVT
VSSGGGGSGGGGSGGGGSDVVMTQSPLSLPVTPGEPASISCRSSQSLLHSNGYNYLDWYLQ
KPGQSPQLLIYLGSNRASGVPDRFSGSGSGTDFTLKISRVEAEDVGVYYCMQALQTPLTFGP
GTKVDIKTTTPAPRPPTPAPTIASQPLSLRPEACRPAAGGAVHTRGLDFACDIYIWAPLAGTC
GVLLLSLVITLYCCWLTKKKYSSSVHDPNGEYMFMRAVNTAKKSRLTDVTLKRGRKKLLYI
FKQPFMRPVQTTQEEDGCSCRFPEEEEGGCELRVKFSRSADAPAYQQGQNQLYNELNLGRR
EEYDVLDKRRGRDPEMGGKPQRRKNPQEGLYNELQKDKMAEAYSEIGMKGERRRGKGH
DGLYQGLSTATKDTYDALHMQALPPR (SEQ ID NO: 105).

Secifically, the chimeric antigen receptor D61-CAR comprises the amino acid sequence shown in SEQ ID NO:106.

EVQLVQSGSEVKKPGSSVKVSCKAPGGTFSSYSVSWVRQAPGQGLEWMGGIIPTFGRT
NYAQKFQGRVTISAHESTGTAYMELSSLRSEDTAVYYCASVRKPYYGMDVWGQGTMVTVS
SGGGGSGGGGSGGGGSAIWMTQSPSSLSASVGDRVTITCRASQSIGRFLNWYQQKPGTAPK
LLISGASRLQGGIPSRFSGSGSGTDFTLTISSLQPEDFATYYCQQSYSTPPTFGQGTKVEIKTTT
PAPRPPTPAPTIASQPLSLRPEACRPAAGGAVHTRGLDFACDIYIWAPLAGTCGVLLLSLVITL
YCCWLTKKKYSSSVHDPNGEYMFMRAVNTAKKSRLTDVTLKRGRKKLLYIFKQPFMRPVQ
TTQEEDGCSCRFPEEEEGGCELRVKFSRSADAPAYQQGQNQLYNELNLGRREEYDVLDKRR
GRDPEMGGKPQRRKNPQEGLYNELQKDKMAEAYSEIGMKGERRRGKGHDGLYQGLSTAT
KDTYDALHMQALPPR (SEQ ID NO: 106).

Secifically, the chimeric antigen receptor D70-CAR comprises the amino acid sequence shown in SEQ ID NO:107.

QVQLVQSGSELKKPGASVKISCKASGYTLTSYGINWVRQAPGKGLELMGWINTKTGN
PTYVQGFTGQVVFSFDTSVNTAYLQINNLKSDDTAVYFCARDRNPYGMDVWGQGTTVTVS
SGGGGSGGGGSGGGGSEIVLTQSPLSLPVTPGEPASISCRSSQSLLHSNGYNYLDWYLQKPG
QSPQLLIYLGSNRASGVPDRFSGSGSGTDFTLKISRVEAEDVGVYYCMQPLQALYTFGQGT
KVEIKTTTPAPRPPTPAPTIASQPLSLRPEACRPAAGGAVHTRGLDFACDIYIWAPLAGTCGV
LLLSLVITLYCCWLTKKKYSSSVHDPNGEYMFMRAVNTAKKSRLTDVTLKRGRKKLLYIFK
QPFMRPVQTTQEEDGCSCRFPEEEEGGCELRVKFSRSADAPAYQQGQNQLYNELNLGRREE
YDVLDKRRGRDPEMGGKPQRRKNPQEGLYNELQKDKMAEAYSEIGMKGERRRGKGHDG
LYQGLSTATKDTYDALHMQALPPR (SEQ ID NO: 107).

Secifically, the chimeric antigen receptor D72-CAR comprises the amino acid sequence shown in SEQ ID NO:108.

QVQLVQSGAEVKKPGSSVKVSCKASGGTFSSYAISWVRQAPGQGLEWMGGIIPIFGTA
NYAQKFQGRVTITADESTSTAYMELSSLRSEDTAVYYCARTGGDYYYYGMDVWGQGTTVT
VSSGGGGSGGGGSGGGGSAIWMTQSPSSLSASVGDRVTITCRASQTISTYLNWYQQKPGKA
PKLLIYGASSLQTGVPSRFSGSGSGTDFTLTISSLQPEDFATYYCQQSYSTPPTFGQGTKVDIK
TTTPAPRPPTPAPTIASQPLSLRPEACRPAAGGAVHTRGLDFACDIYIWAPLAGTCGVLLLSLV
ITLYCCWLTKKKYSSSVHDPNGEYMFMRAVNTAKKSRLTDVTLKRGRKKLLYIFKQPFMRP
VQTTQEEDGCSCRFPEEEEGGCELRVKFSRSADAPAYQQGQNQLYNELNLGRREEYDVLD
KRRGRDPEMGGKPQRRKNPQEGLYNELQKDKMAEAYSEIGMKGERRRGKGHDGLYQGLS
TATKDTYDALHMQALPPR (SEQ ID NO: 108).

Secifically, the chimeric antigen receptor D76-CAR comprises the amino acid sequence shown in SEQ ID NO:156.

MGVKVLFALICIAVAEAEVQLVESGAEVKKPGASVKVSCKASGYTFTDYFLHWVRQA
PGQGLEWMGWINPNSGGTNYAQKFQGWVTMTRDTSISTAYLELSSLTSDDTAVYYCAVDP
FDYWGQGTLVTVSSGGGGSGGGGSGGGGSDVVMTQSPLSSPVTLGQPASISCRSSQSLVHS
DGNTYLSWLHQRPGQPPRLLIHKISNRFSGVPDRFSGSGAGTDFTLKISRVEAEDVGVFYCM
QATQFPITFGQGTRLEIKTTTPAPRPPTPAPTIASQPLSLRPEACRPAAGGAVHTRGLDFACDI
YIWAPLAGTCGVLLLSLVITLYCCWLTKKKYSSSVHDPNGEYMFMRAVNTAKKSRLTDVTL
KRGRKKLLYIFKQPFMRPVQTTQEEDGCSCRFPEEEEGGCELRVKFSRSADAPAYQQGQNQ
LYNELNLGRREEYDVLDKRRGRDPEMGGKPQRRKNPQEGLYNELQKDKMAEAYSEIGMK
GERRRGKGHDGLYQGLSTATKDTYDALHMQALPPR (SEQ ID NO: 156).

Secifically, the chimeric antigen receptor D79-CAR comprises the amino acid sequence shown in SEQ ID NO:157.

MGVKVLFALICIAVAEAQVQLVQSGAEVKKPGASVKVSCKASGYTFTGYYMHWVRQ
APGQGLEWMGWINPNSGGTNYAQKFQGRVTMTRDTSISTAYMELSRLRSDDTAVYYCARV
GGSYYGGFDYWGQGTLVTVSSGGGGSGGGGSGGGGSEIVLTQSPLSLPVTPGEPASISCRSS

QSLLHSNGYNYLDWYLQKPGQSPQLLMYLSSNRASGVPDRFSGSGSGRDFTLKISRVEAED
VGLYYCMQALQTPPTFGQGTRLEIKTTTPAPRPPTPAPTIASQPLSLRPEACRPAAGGAVHTR
GLDFACDIYIWAPLAGTCGVLLLSLVITLYCCWLTKKKYSSSVHDPNGEYMFMRAVNTAKK
SRLTDVTLKRGRKKLLYIFKQPFMRPVQTTQEEDGCSCRFPEEEEGGCELRVKFSRSADAPA
YQQGQNQLYNELNLGRREEYDVLDKRRGRDPEMGGKPQRRKNPQEGLYNELQKDKMAE
AYSEIGMKGERRRGKGHDGLYQGLSTATKDTYDALHMQALPPR (SEQ ID NO: 157).

Secifically, the chimeric antigen receptor D85-CAR comprises the amino acid sequence shown in SEQ ID NO:158.

MGVKVLFALICIAVAEAQVQLVQSGAEVKKPGASVKVSCKASGYTFTGYYMHWVRQ
APGQGLEWMGWINPNSGGTNYAQKFQGRVTMTRDTSISTAYMELSRLRSDDTAVYYCASD
PFDYWGQGTLVTVSSGGGGSGGGGSGGGGSDVVMTQSPLSSPVTLGQPASISCRSSQSLVY
SDGNTYLSWLQQRPGQPPRLLIYKVSNRFSGVPDRFSGSGAGTDFTLTISRVEAEDVGVYY
CMQATQYPLAFGGGTKLEIKTTTPAPRPPTPAPTIASQPLSLRPEACRPAAGGAVHTRGLDFA
CDIYIWAPLAGTCGVLLLSLVITLYCCWLTKKKYSSSVHDPNGEYMFMRAVNTAKKSRLTD
VTLKRGRKKLLYIFKQPFMRPVQTTQEEDGCSCRFPEEEEGGCELRVKFSRSADAPAYQQG
QNQLYNELNLGRREEYDVLDKRRGRDPEMGGKPQRRKNPQEGLYNELQKDKMAEAYSEI
GMKGERRRGKGHDGLYQGLSTATKDTYDALHMQALPPR (SEQ ID NO: 158).

Secifically, the chimeric antigen receptor D103-CAR comprises the amino acid sequence shown in SEQ ID NO:159.

MGVKVLFALICIAVAEAQVQLVQSGAEVKKPGESLKISCKGSGYSFTNYWIGWVRQMP
GKGLEWMGIIYPGDSDTRYSPSFQGQVTISADKSISTAYLQWSSLKASDTAMYYCARPQGA
FDIWGQGTMVTVSSGGGGSGGGGSGGGGSEIVMTQTPLSLPVTLGQPASISCRSSQSLVYSD
GNTYLNWFQQRPGQSPRRLIYKVSNRDSGVPDRFSGSGSGTDFTLKISRVEAEDVGVYYCM
QGTHWPLTFGQGTKVEIKTTTPAPRPPTPAPTIASQPLSLRPEACRPAAGGAVHTRGLDFACD
IYIWAPLAGTCGVLLLSLVITLYCCWLTKKKYSSSVHDPNGEYMFMRAVNTAKKSRLTDVT
LKRGRKKLLYIFKQPFMRPVQTTQEEDGCSCRFPEEEEGGCELRVKFSRSADAPAYQQGQN
QLYNELNLGRREEYDVLDKRRGRDPEMGGKPQRRKNPQEGLYNELQKDKMAEAYSEIGM
KGERRRGKGHDGLYQGLSTATKDTYDALHMQALPPR (SEQ ID NO: 159).

In some embodiments, the chimeric antigen receptor further comprises a restriction enzyme cleavage site.

Specifically, the restriction enzyme cleavage site is selected from BamHI, SpeI, SalI, KpnI, XhoI, SphI, XbaI, EcoRI, and the like.

Specifically, the restriction enzyme cleavage site is located at the N-terminus of the hinge region and at the C-terminus of the extracellular antigen-binding structure. In some embodiments, the amino acid sequence of the EcoRI restriction enzyme cleavage site is: EF.

As a specific example, the chimeric antigen receptor D9-CAR comprising a restriction enzyme cleavage site comprises the amino acid sequence shown in SEQ ID NO:125:
EVHLVESGGGVVQPGRSLRLSCAASGFTFSNYAMHWVRQAPGKGLEWVAVISYDGSK KYYADSVKGRFTISRDDSKNTLYLQMNSLRAEDTAVYYCAKPAGSTSGFDYWGQGTLVTV SSGGGGSGGGGSGGGGSQSALTQPASVSGSPGQSITISCTGTSSDVGGYNYVSWYQQHPGK APKLMIYDVSNRPSGVSNRFSGSKSGNTASLTISGLQAEDEADYYCSSYTSSSTLVVFGGGT KLTVL**EF**TTTPAPRPPTPAPTIASQPLSLRPEACRPAAGGAVHTRGLDFACDIYIWAPLAGTC GVLLLSLVITLYCCWLTKKKYSSSVHDPNGEYMFMRAVNTAKKSRLTDVTLKRGRKKLLYI FKQPFMRPVQTTQEEDGCSCRFPEEEEGGCELRVKFSRSADAPAYQQGQNQLYNELNLGRR EEYDVLDKRRGRDPEMGGKPQRRKNPQEGLYNELQKDKMAEAYSEIGMKGERRRGKGH DGLYQGLSTATKDTYDALHMQALPPR (SEQ ID NO: 125), wherein the bolded portion represents the amino acid sequence of the EcoRI restriction enzyme cleavage site;

The chimeric antigen receptors described in the present invention may all comprise an amino acid sequence containing a restriction enzyme cleavage site as shown in the above specific examples, the restriction enzyme cleavage site is located at the N-terminus of the hinge region and at the C-terminus of the extracellular antigen-binding structure.

In some embodiments, the chimeric antigen receptor further comprises a signal peptide covalently linked to the N-terminus of the extracellular antigen-binding domain.

In some embodiments, the signal peptide comprises the amino acid sequence shown in SEQ ID NO:91.
MGVKVLFALICIAVAEA (SEQ ID NO: 91).

In another aspect, the present invention provides an isolated nucleic acid molecule comprising a nucleotide sequence encoding the chimeric antigen receptor described herein.

An isolated nucleic acid refers to a nucleic acid molecule that has been separated from components of its natural environment.

In some embodiments, the nucleic acid molecule is intended to include DNA molecules and RNA molecules. Nucleic acid molecules may be single-stranded or double-stranded and may be cDNA.

In some embodiments, the nucleic acid molecule comprises the nucleotide sequence shown in any one of SEQ ID NO: 109-118 or SEQ ID NO: 160-163.

Secifically, the chimeric antigen receptor D9-CAR comprises the nucleotide sequence shown in SEQ ID NO:109.

GAGGTGCATCTGGTGGAGTCTGGGGGAGGCGTGGTCCAGCCTGGGAGGTCCCTGA
GACTCTCCTGTGCAGCCTCTGGATTCACCTTCAGTAACTATGCTATGCACTGGGTCCGCC
AGGCTCCAGGCAAGGGGCTGGAGTGGGTGGCAGTTATCTCATATGATGGAAGTAAAAA
TACTACGCAGACTCCGTGAAGGGCCGATTCACCATCTCCAGAGACGATTCCAAGAACAC
GCTGTATCTGCAAATGAACAGCCTGAGAGCCGAGGACACGGCCGTATATTACTGTGCGA
AACCCGCTGGTAGTACCAGTGGGTTTGACTACTGGGGCCAGGGAACCCTGGTCACCGTC
TCCTCAGGTGGCGGCGGTTCAGGCGGAGGTGGCTCTGGCGGTGGCGGATCGCAGTCTG

CCCTGACTCAGCCTGCCTCCGTGTCTGGGTCTCCTGGACAGTCGATCACCATCTCCTGCA
CTGGAACCAGCAGTGACGTTGGTGGTTATAACTATGTCTCCTGGTACCAACAACACCCA
GGCAAAGCCCCCAAACTCATGATTTATGATGTCAGTAATCGGCCCTCAGGGGTTTCTAAT
CGCTTCTCTGGCTCCAAGTCTGGCAACACGGCCTCCCTGACCATCTCTGGGCTCCAGGC
TGAGGACGAGGCTGATTATTACTGCAGCTCATATACAAGCAGCAGCACTCTCGTGGTATT
CGGCGGAGGGACCAAGCTGACCGTCCTAACCACGACGCCAGCGCCGCGACCACCAACA
CCGGCGCCCACCATCGCGTCGCAGCCCCTGTCCCTGCGCCCAGAGGCGTGCCGGCCAG
CGGCGGGGGGCGCAGTGCACACGAGGGGGCTGGACTTCGCCTGTGATATCTACATCTGG
GCGCCCTTGGCCGGGACTTGTGGGGTCCTTCTCCTGTCACTGGTTATCACCCTTTACTGC
TGTTGGCTTACAAAAAAGAAGTATTCATCCAGTGTGCACGACCCTAACGGTGAATACAT
GTTCATGAGAGCAGTGAACACAGCCAAAAAATCTAGACTCACAGATGTGACCCTAAAG
CGCGGCCGCAAGAAGCTGCTGTACATCTTCAAGCAGCCCTTCATGCGCCCCGTGCAGAC
CACCCAGGAGGAGGACGGCTGTAGCTGCCGCTTCCCCGAGGAGGAGGAGGGCGGCTG
CGAGCTGCGCGTGAAATTTAGCCGCAGCGCCGACGCCCCCGCCTACCAGCAGGGCCAG
AACCAGCTGTACAACGAGCTGAACCTGGGCCGCCGCGAGGAGTACGACGTGCTGGACA
AGCGCCGGGGCCGCGACCCCGAGATGGGCGGCAAGCCCAGCGCCGCAAGAACCCCC
AGGAGGGCCTGTACAACGAGCTGCAGAAGGACAAGATGGCCGAAGCCTACAGCGAGAT
CGGCATGAAGGGCGAGCGCCGCCGGGGCAAGGGCCACGACGGCCTGTACCAGGGCCT
GAGCACCGCCACCAAGGACACCTACGACGCCCTGCACATGCAGGCCCTGCCCCCCCGC
(SEQ ID NO: 109).

Secifically, the chimeric antigen receptor D10-CAR comprises the nucleotide sequence shown in SEQ ID NO:110.

CAGGTGCAGCTGGTGCAATCTGGGGGAGGTGTGGTACGGCCTGGGGGGTCCCTGA
GACTCTCCTGTGCAGCCTCTGGATTCACCTTTGATGATTATGGCATGAGCTGGGTCCGCC
AAGCTCCAGGGAAGGGGCTGGAGTGGGTCTCTGGTATTAATTGGAATGGTGGTAGCACA
GGTTATGCAGACTCTGTGAAGGGCCGATTCACCATCTCCAGAGACAATTCCAAGAACAC
GCTGTATCTGCAAATGAACAGCCTGAGAGCTGAGGACACGGCTGTCTATTACTGTGCGA
CTAGCAGCGTCAAGCACTACTGGGGCCAGGGAACCCTGGTCACTGTCTCCTCAGGTGG
CGGCGGTTCAGGCGGAGGTGGCTCTGGCGGTGGCGGATCGCAGCCTGTGCTGACTCAG
CCACCCTCAGCGTCTGCGACCCCCGGACAGAGGGTCACCATCTCCTGCTCTGGAAGCA
GCTCCAACATCGGGACTAATACTGTGAACTGGTACCAGCAGGTCCCAGGAACGGCCCCC
AAAGTCCTCATCTACAGGAATGATCTGCGGCCCTCAGGGGTCCCTGACCGATTCTCTGG
CTCCAAGTCTGGCACCTCAGCCTCCCTGGCCATCAGTGGGCTCCAGTCTGACGATGAGG
CTGATTATTACTGTGCAGCTTGGGATGACAGCCTGAATGGTGTGGTTTTCGGCGGAGGG
ACCAAGGTCACCGTCCTAACCACGACGCCAGCGCCGCGACCACCAACACCGGCGCCCA
CCATCGCGTCGCAGCCCCTGTCCCTGCGCCCAGAGGCGTGCCGGCCAGCGGCGGGGGG
CGCAGTGCACACGAGGGGGCTGGACTTCGCCTGTGATATCTACATCTGGGCGCCCTTGG

CCGGGACTTGTGGGGTCCTTCTCCTGTCACTGGTTATCACCCTTTACTGCTGTTGGCTTA
CAAAAAAGAAGTATTCATCCAGTGTGCACGACCCTAACGGTGAATACATGTTCATGAGA
GCAGTGAACACAGCCAAAAAATCTAGACTCACAGATGTGACCCTAAAGCGCGGCCGCA
AGAAGCTGCTGTACATCTTCAAGCAGCCCTTCATGCGCCCCGTGCAGACCACCCAGGAG
GAGGACGGCTGTAGCTGCCGCTTCCCCGAGGAGGAGGAGGGCGGCTGCGAGCTGCGCG
TGAAATTTAGCCGCAGCGCCGACGCCCCCGCCTACCAGCAGGGCCAGAACCAGCTGTA
CAACGAGCTGAACCTGGGCCGCCGCGAGGAGTACGACGTGCTGGACAAGCGCCGGGG
CCGCGACCCCGAGATGGGCGGCAAGCCCCAGCGCCGCAAGAACCCCCAGGAGGGCCT
GTACAACGAGCTGCAGAAGGACAAGATGGCCGAAGCCTACAGCGAGATCGGCATGAAG
GGCGAGCGCCGCCGGGGCAAGGGCCACGACGGCCTGTACCAGGGCCTGAGCACCGCC
ACCAAGGACACCTACGACGCCCTGCACATGCAGGCCCTGCCCCCCCGC (SEQ ID NO:
110).

Secifically, the chimeric antigen receptor D28-CAR comprises the nucleotide sequence shown in SEQ ID NO:111.

CAGGTGCAGCTGGTGCAATCTGGACCTGAGATGAAGAAGCCTGGGGCCTCAGTGA
AGGTCGCCTGCAAAACTTCTGGTTACATTTTATCAACTATGGTATCACATGGGTGCGAC
AGGCCCCTGGACAAGGACTTGAGTGGATGGGCTGGATCAGCCCTTACAATGGTCACACA
ATCTATGCACAGAAGGTCCAGGGCAGAGGCACTATGACCACAGAGACTCCCACGAGCA
CAGCCTACCTGGACCTAACCAATCTGACACCTGACGACACGGCCATCTATTACTGTGCG
ACTGGACTAAACTCGCGACTGCAATACTGGGGCCAGGGAGCCCTGGTCACCGTCTCCTC
AGGTGGCGGCGGTTCAGGCGGAGGTGGCTCTGGCGGTGGCGGATCGCAGCCTGTGCTG
ACTCAGCCTGCCTCCGTGTCTGGGTCTCCTGGACAGTCGATCACCATCTCCTGCACTGG
AACCAGCAGTGACGTTGGTGGTTATAACTATGTCTCCTGGTACCAACAGCACCCAGGCA
AAGCCCCCAAACTCATGATTTATGATGTCAGTAAGCGGCCCTCAGGGGTTTCTAATCGCT
TCTCTGGCTCCAAGTCTGGCAACACGGCCTCCCTGACCATCTCTGGGCTCCAGGCTGAG
GACGAGGCTGATTATTACTGCAGCTCATATACAAGCAGCAGCACCGATGTCTTCGGAACT
GGGACCAAGGTCACCGTCCTAACCACGACGCCAGCGCCGCGACCACCAACACCGGCGC
CCACCATCGCGTCGCAGCCCCTGTCCCTGCGCCCAGAGGCGTGCCGGCCAGCGGCGGG
GGCGCAGTGCACACGAGGGGGCTGGACTTCGCCTGTGATATCTACATCTGGGCGCCCT
TGGCCGGGACTTGTGGGGTCCTTCTCCTGTCACTGGTTATCACCCTTTACTGCTGTTGGC
TTACAAAAAAGAAGTATTCATCCAGTGTGCACGACCCTAACGGTGAATACATGTTCATGA
GAGCAGTGAACACAGCCAAAAAATCTAGACTCACAGATGTGACCCTAAAGCGCGGCCG
CAAGAAGCTGCTGTACATCTTCAAGCAGCCCTTCATGCGCCCCGTGCAGACCACCCAGG
AGGAGGACGGCTGTAGCTGCCGCTTCCCCGAGGAGGAGGAGGGCGGCTGCGAGCTGC
GCGTGAAATTTAGCCGCAGCGCCGACGCCCCCGCCTACCAGCAGGGCCAGAACCAGCT
GTACAACGAGCTGAACCTGGGCCGCCGCGAGGAGTACGACGTGCTGGACAAGCGCCGG
GGCCGCGACCCCGAGATGGGCGGCAAGCCCCAGCGCCGCAAGAACCCCCAGGAGGGC

CTGTACAACGAGCTGCAGAAGGACAAGATGGCCGAAGCCTACAGCGAGATCGGCATGA
AGGGCGAGCGCCGCCGGGGCAAGGGCCACGACGGCCTGTACCAGGGCCTGAGCACCG
CCACCAAGGACACCTACGACGCCCTGCACATGCAGGCCCTGCCCCCCCGC (SEQ ID NO:
111).

Secifically, the chimeric antigen receptor D30-CAR comprises the nucleotide sequence shown in SEQ ID NO:112.

GAAGTGCAGCTGGTGCAGTCTGGGGCTGAGGTGAAGAAGCCTGGGGCCTCAATGA
AGGTCTCCTGCAAGGCTTCTGGATACACCTTCACCGGCTACTATATGCACTGGGTGCGAC
AGGCCCCTGGACAAGGGCTTGAGTGGATGGGATGGATCAACCCTGTGAGTGGTGGCAC
AAACTATGCACAGAAATTTCAGGGCAGAGTCACCATGACCAGGGACACGTCCATCGGC
ACAGCCTACATGGAGTTGACTGGGCTGAGATCTGACGACACGGCCGTATATTATTGTGCG
GCAGATCCTTTTGATATCTGGGGCCAAGGGACAATGGTCACCGTCTCCTCAGGTGGCGG
CGGTTCAGGCGGAGGTGGCTCTGGCGGTGGCGGATCGCAGTCTGCCCTGACTCAGCCT
GCCTCCGTGTCTGGGTCTCCTGGACAGTCGATCACCATCTCCTGCACTGGAACCAGCAG
TGACGTTGGTGGTTATAACTATGTCTCCTGGTACCAACAGCACCCAGGCAAAGCCCCCA
AACTCATGATTTATGATGTCAGTAAGCGGCCCTCAGGGGTTTCCAATCGCTTCTCTGGCT
CCAAGTCTGGCAACACGGCCTCCCTGACCATCTCTGGGCTCCAGGCTGAGGACGAGGC
TGATTATTACTGCAGCTCATATACAAGCAGGAGCACCCCTGGTTATGTCTTCGGAACTGG
GACCAAGCTGACCGTCCTAACCACGACGCCAGCGCCGCGACCACCAACACCGGCGCCC
ACCATCGCGTCGCAGCCCCTGTCCCTGCGCCCAGAGGCGTGCCGGCCAGCGGCGGGG
GCGCAGTGCACACGAGGGGGCTGGACTTCGCCTGTGATATCTACATCTGGGCGCCCTTG
GCCGGGACTTGTGGGGTCCTTCTCCTGTCACTGGTTATCACCCTTTACTGCTGTTGGCTT
ACAAAAAAGAAGTATTCATCCAGTGTGCACGACCCTAACGGTGAATACATGTTCATGAG
AGCAGTGAACACAGCCAAAAAATCTAGACTCACAGATGTGACCCTAAAGCGCGGCCGC
AAGAAGCTGCTGTACATCTTCAAGCAGCCCTTCATGCGCCCCGTGCAGACCACCCAGGA
GGAGGACGGCTGTAGCTGCCGCTTCCCCGAGGAGGAGGAGGGCGGCTGCGAGCTGCGC
GTGAAATTTAGCCGCAGCGCCGACGCCCCCGCCTACCAGCAGGGCCAGAACCAGCTGT
ACAACGAGCTGAACCTGGGCCGCCGCGAGGAGTACGACGTGCTGGACAAGCGCCGGG
GCCGCGACCCCGAGATGGGCGGCAAGCCCCAGCGCCGCAAGAACCCCCAGGAGGGCC
TGTACAACGAGCTGCAGAAGGACAAGATGGCCGAAGCCTACAGCGAGATCGGCATGAA
GGGCGAGCGCCGCCGGGGCAAGGGCCACGACGGCCTGTACCAGGGCCTGAGCACCGC
CACCAAGGACACCTACGACGCCCTGCACATGCAGGCCCTGCCCCCCGC (SEQ ID NO:
112).

Secifically, the chimeric antigen receptor D32-CAR comprises the nucleotide sequence shown in SEQ ID NO:113.

CAGGTCCAGCTGGTGCAATCTGGAGCTGAGGTGAAGAAGCCTGGGGCCTCAGTGA
AGGTCTCCTGCAAGGCCTCTGGTTACACCTTTACCAACTATGGTTTCAGCTGGGTGCGAC

AGGCCCCTGGACAAGGGCTTGAGTGGATGGATGGATCAGCTCTTATAATGGCAACACA
AAGTATGCACAGAAGTTCCGGGCAGAGTCACCATGACCACAGACACATCCACGAGCA
CAGGCTACATGGAACTGAGGAGCCTGAGATCTGACGACACGGCCGTGTATTACTGTGCG
AGGGGCGGGGACTGGCCCGCGGACTACTGGGGCCAGGGAACCCTGGTCACTGTCTCCT
CAGGTGGCGGCGGTTCAGGCGGAGGTGGCTCTGGCGGTGGCGGATCGCAGGCTGTGGT
GACTCAGGAGCCATCGTTCTCAGTGTCCCCTGGAGGGACAGTCACACTCACTTGTGGCT
TGAGCTCTGGCTCAGTTAGTAACAATTATTACCCCAGCTGGTACCAGCAGATCCCAGGCC
AGGCTCCACGCACGCTCATCTACAGCACAAATACTCGCTCTTCTGGGGTCCCTGATCGCT
TCTCTGGCTCCATCCTTGGGAACAAAGCTGCCCTCACCATCACGGGGGCCCAGGCAGAT
GATGAAGCTGATTATTACTGTGTGGTGTATATGGGTAGTGGCATTTGGGTGTTCGGCGGA
GGGACCAAGCTGACCGTCCTAACCACGACGCCAGCGCCGCGACCACCAACACCGGCGC
CCACCATCGCGTCGCAGCCCCTGTCCCTGCGCCCAGAGGCGTGCCGGCCAGCGGCGG
GGGCGCAGTGCACACGAGGGGGCTGGACTTCGCCTGTGATATCTACATCTGGGCGCCCT
TGGCCGGGACTTGTGGGGTCCTTCTCCTGTCACTGGTTATCACCCTTTACTGCTGTTGGC
TTACAAAAAGAAGTATTCATCCAGTGTGCACGACCCTAACGGTGAATACATGTTCATGA
GAGCAGTGAACACAGCCAAAAAATCTAGACTCACAGATGTGACCCTAAAGCGCGGCCG
CAAGAAGCTGCTGTACATCTTCAAGCAGCCCTTCATGCGCCCCGTGCAGACCACCCAGG
AGGAGGACGGCTGTAGCTGCCGCTTCCCCGAGGAGGAGGAGGGCGGCTGCGAGCTGC
GCGTGAAATTTAGCCGCAGCGCCGACGCCCCCGCCTACCAGCAGGGCCAGAACCAGCT
GTACAACGAGCTGAACCTGGGCCGCCGCGAGGAGTACGACGTGCTGGACAAGCGCCGG
GGCCGCGACCCCGAGATGGGCGGCAAGCCCCAGCGCCGCAAGAACCCCCAGGAGGGC
CTGTACAACGAGCTGCAGAAGGACAAGATGGCCGAAGCCTACAGCGAGATCGGCATGA
AGGGCGAGCGCCGCCGGGGCAAGGGCCACGACGGCCTGTACCAGGGCCTGAGCACCG
CCACCAAGGACACCTACGACGCCCTGCACATGCAGGCCCTGCCCCCCCGC (SEQ ID NO:
113).

Secifically, the chimeric antigen receptor D46-CAR comprises the nucleotide sequence shown in SEQ ID NO:114.

CAGGTGCAGCTGGTGCAATCTGGGGCTGAGGTGAAGAAGCCTGGGGCCTCAGTGA
AGGTCTCCTGCAAGGCTTCTGGTTACACCTTTACCAGCTATGGTATCAGCTGGGTGCGAC
AGGCCCCTGGACAAGGGCTTGAGTGGATGGGATGGATCAACCCTAACAGTGGTGGCAC
AAACTATGCACAGAAGTTTCAGGGCAGGGTCACCATGACCAGGGACACGTCCATCAGC
ACAGCCTACATGGAGCTGAGCAGGCTGAGATCTGACGACACGGCCGTGTATTACTGTGC
GAGAGCTGGGGATCTTCGGGACTACTACTACTACGGTATGGACGTCTGGGGCCAAGGGA
CCACGGTCACCGTCTCCTCAGGTGGCGGCGGTTCAGGCGGAGGTGGCTCTGGCGGTGG
CGGATCGCAGTCTGTGTTGACGCAGCCGCCCTCAGTGTCTGGGGCCCCAGGGCAGAGG
GTCACCATCTCCTGCACTGGGAGCAGCTCCAACATCGGGGCAGGTTATGATGTACACTG
GTACCAGCAGCTTCCAGGAACAGCCCCCAAACTCCTCATCTATGGTAACAGCAATCGGC

CCTCAGGGGTCCCTGACCGATTCTCTGGCTCCAAGTCTGGCACCTCAGCCTCCCTGGCC
ATCACTGGGCTCCAGGCTGAGGATGAGGCTGATTATTACTGCCAGTCCTATGACAGCAGC
CTGAGTGGTTGGGTGTTCGGCGGAGGCACCCAGCTGACCGCCCTCACCACGACGCCAG
CGCCGCGACCACCAACACCGGCGCCCACCATCGCGTCGCAGCCCCTGTCCCTGCGCCC
AGAGGCGTGCCGGCCAGCGGCGGGGGGCGCAGTGCACACGAGGGGGCTGGACTTCGC
CTGTGATATCTACATCTGGGCGCCCTTGGCCGGGACTTGTGGGGTCCTTCTCCTGTCACT
GGTTATCACCCTTTACTGCTGTTGGCTTACAAAAAGAAGTATTCATCCAGTGTGCACGA
CCCTAACGGTGAATACATGTTCATGAGAGCAGTGAACACAGCCAAAAAATCTAGACTCA
CAGATGTGACCCTAAAGCGCGGCCGCAAGAAGCTGCTGTACATCTTCAAGCAGCCCTTC
ATGCGCCCCGTGCAGACCACCCAGGAGGAGGACGGCTGTAGCTGCCGCTTCCCCGAGG
AGGAGGAGGGCGGCTGCGAGCTGCGCGTGAAATTTAGCCGCAGCGCCGACGCCCCCGC
CTACCAGCAGGGCCAGAACCAGCTGTACAACGAGCTGAACCTGGGCCGCCGCGAGGAG
TACGACGTGCTGGACAAGCGCCGGGGCCGCGACCCCGAGATGGGCGGCAAGCCCCAGC
GCCGCAAGAACCCCCAGGAGGGCCTGTACAACGAGCTGCAGAAGGACAAGATGGCCG
AAGCCTACAGCGAGATCGGCATGAAGGGCGAGCGCCGCCGGGGCAAGGGCCACGACG
GCCTGTACCAGGGCCTGAGCACCGCCACCAAGGACACCTACGACGCCCTGCACATGCA
GGCCCTGCCCCCCCGC (SEQ ID NO: 114).

Secifically, the chimeric antigen receptor D59-CAR comprises the nucleotide sequence shown in SEQ ID NO:115.

CAGGTGCAGCTGGTGCAATCTGGGGCTGAGGTGAAGAAGCCTGGGGCCTCAGTGA
AAGTTTCCTGCAAGGCATCTGGATACACCTTCACCAATCACTTCATACACTGGGTGCGGC
AGGCCCCTGGACAAGGGCTTGAGTGGATGGGATGGATCAGCGCTAACAGTGGAAACAC
AGACTATGCACAGAAGTTCCAGGGCAGAGTCACCATGACCACAGACACATCCACGAGC
ACAGCCTACATGGAGCTGAGGAGCCTGAGATATGACGACACGGCCGTGTATTACTGTGC
GAGAGATCTTGCAACCGTGGATGGTTTTGATATCTGGGGCCAAGGGACAATGGTCACCG
TCTCTTCAGGTGGCGGCGGTTCAGGCGGAGGTGGCTCTGGCGGTGGCGGATCGGATGTT
GTGATGACTCAGTCTCCACTCTCCCTGCCCGTCACCCCTGGAGAGCCGGCCTCCATCTC
CTGCAGGTCTAGTCAGAGCCTCCTGCATAGTAATGGATACAACTATTTGGATTGGTACCT
GCAGAAGCCAGGGCAGTCTCCACAGCTCCTGATCTATTTGGGTTCTAATCGGGCCTCCG
GGGTCCCTGACAGGTTCAGTGGCAGTGGATCAGGCACAGATTTTACACTGAAAATCAGC
AGAGTGGAGGCTGAGGATGTTGGGGTTTATTACTGCATGCAAGCTCTACAAACTCCTCT
CACTTTCGGCCCTGGGACCAAAGTGGATATCAAAACCACGACGCCAGCGCCGCGACCA
CCAACACCGGCGCCCACCATCGCGTCGCAGCCCCTGTCCCTGCGCCCAGAGGCGTGCC
GGCCAGCGGCGGGGGGCGCAGTGCACACGAGGGGGCTGGACTTCGCCTGTGATATCTA
CATCTGGGCGCCCTTGGCCGGGACTTGTGGGGTCCTTCTCCTGTCACTGGTTATCACCCT
TTACTGCTGTTGGCTTACAAAAAAGAAGTATTCATCCAGTGTGCACGACCCTAACGGTG
AATACATGTTCATGAGAGCAGTGAACACAGCCAAAAAATCTAGACTCACAGATGTGACC

CTAAAGCGCGGCCGCAAGAAGCTGCTGTACATCTTCAAGCAGCCCTTCATGCGCCCCGT
GCAGACCACCCAGGAGGAGGACGGCTGTAGCTGCCGCTTCCCCGAGGAGGAGGAGGG
CGGCTGCGAGCTGCGCGTGAAATTTAGCCGCAGCGCCGACGCCCCCGCCTACCAGCAG
GGCCAGAACCAGCTGTACAACGAGCTGAACCTGGGCCGCCGCGAGGAGTACGACGTGC
TGGACAAGCGCCGGGGCCGCGACCCCGAGATGGGCGGCAAGCCCCAGCGCCGCAAGA
ACCCCCAGGAGGGCCTGTACAACGAGCTGCAGAAGGACAAGATGGCCGAAGCCTACAG
CGAGATCGGCATGAAGGGCGAGCGCCGCCGGGGCAAGGGCCACGACGGCCTGTACCAG
GGCCTGAGCACCGCCACCAAGGACACCTACGACGCCCTGCACATGCAGGCCCTGCCCC
CCCGC (SEQ ID NO: 115).

Secifically, the chimeric antigen receptor D61-CAR comprises the nucleotide sequence shown in SEQ ID NO:116.

GAGGTCCAGCTGGTACAGTCTGGGTCTGAGGTGAAGAAGCCTGGGTCCTCGGTGA
AGGTCTCCTGCAAGGCTCCTGGAGGCACCTTCAGCAGCTATAGTGTCAGCTGGGTGCGA
CAGGCCCCTGGACAAGGGCTTGAGTGGATGGGAGGGATCATCCCTACCTTTGGTCGAAC
AAACTACGCACAGAAGTTTCAGGGCAGAGTCACAATTAGCGCGCACGAATCCACGGGC
ACGGCCTACATGGAGCTGAGCAGTCTGAGATCTGAGGACACGGCCGTCTATTACTGTGC
GAGCGTCCGGAAGCCCTACTACGGTATGGACGTCTGGGGCCAAGGGACAATGGTCACC
GTCTCTTCAGGTGGCGGCGGTTCAGGCGGAGGTGGCTCTGGCGGTGGCGGATCGGCCA
TCTGGATGACCCAGTCTCCATCCTCCCTGTCTGCATCTGTAGGAGACAGAGTCACCATCA
CTTGCCGGGCAAGTCAGAGCATTGGTAGGTTTTTAAATTGGTATCAGCAGAAACCAGGG
ACAGCCCCTAAACTCCTGATCTCTGGTGCATCCCGTTTGCAAGGTGGAATCCCATCAAG
GTTCAGCGGCAGTGGATCTGGGACAGATTTCACTCTCACCATCAGCAGTCTGCAACCTG
AAGATTTTGCAACTTACTACTGTCAACAGAGTTACAGTACCCCTCCGACGTTCGGCCAA
GGGACCAAGGTGGAAATCAAAACCACGACGCCAGCGCCGCGACCACCAACACCGGCG
CCCACCATCGCGTCGCAGCCCCTGTCCCTGCGCCCAGAGGCGTGCCGGCCAGCGGCGG
GGGGCGCAGTGCACACGAGGGGGCTGGACTTCGCCTGTGATATCTACATCTGGGCGCCC
TTGGCCGGGACTTGTGGGGTCCTTCTCCTGTCACTGGTTATCACCCTTTACTGCTGTTGG
CTTACAAAAAGAAGTATTCATCCAGTGTGCACGACCCTAACGGTGAATACATGTTCATG
AGAGCAGTGAACACAGCCAAAAAATCTAGACTCACAGATGTGACCCTAAAGCGCGGCC
GCAAGAAGCTGCTGTACATCTTCAAGCAGCCCTTCATGCGCCCCGTGCAGACCACCCAG
GAGGAGGACGGCTGTAGCTGCCGCTTCCCCGAGGAGGAGGAGGGCGGCTGCGAGCTG
CGCGTGAAATTTAGCCGCAGCGCCGACGCCCCCGCCTACCAGCAGGGCCAGAACCAGC
TGTACAACGAGCTGAACCTGGGCCGCCGCGAGGAGTACGACGTGCTGGACAAGCGCCG
GGGCCGCGACCCCGAGATGGGCGGCAAGCCCAGCGCCGCAAGAACCCCCAGGAGGG
CCTGTACAACGAGCTGCAGAAGGACAAGATGGCCGAAGCCTACAGCGAGATCGGCATG
AAGGGCGAGCGCCGCCGGGGCAAGGGCCACGACGGCCTGTACCAGGGCCTGAGCACC
GCCACCAAGGACACCTACGACGCCCTGCACATGCAGGCCCTGCCCCCCCGC (SEQ ID

NO: 116).

Secifically, the chimeric antigen receptor D70-CAR comprises the nucleotide sequence shown in SEQ ID NO:117.

CAGGTCCAGCTGGTGCAATCTGGGTCTGAGTTGAAGAAGCCTGGGGCCTCAGTGA
AGATCTCCTGCAAGGCCTCTGGATACACCCTCACAAGTTATGGCATTAATTGGGTGCGAC
AGGCCCCTGGAAAAGGGCTTGAGCTGATGGATGGATCAATACCAAGACTGGAATCC
AACCTACGTCCAGGGCTTCACAGGACAGGTTGTCTTTTCCTTCGACACCTCTGTCAATAC
GGCATATCTGCAGATCAACAATCTAAAGTCTGACGACACTGCCGTGTATTTCTGTGCGAG
AGACCGAAATCCCTACGGTATGGACGTCTGGGGCCAGGGACCACGGTCACCGTCTCCT
CAGGTGGCGGCGGTTCAGGCGGAGGTGGCTCTGGCGGTGGCGGATCGGAAATTGTGCT
GACTCAGTCTCCACTCTCCCTGCCCGTCACCCCTGGAGAGCCGGCCTCCATCTCCTGCA
GGTCTAGTCAGAGCCTCCTGCATAGTAATGGATACAACTATTTGGATTGGTACCTGCAGA
AGCCAGGGCAGTCTCCACAGCTCCTGATCTACTTGGGTTCTAATCGGGCCTCCGGGGTC
CCTGACAGGTTCAGTGGCAGTGGATCAGGCACAGATTTTACACTGAAAATCAGCAGAGT
GGAGGCTGAGGATGTTGGGGTTTATTACTGCATGCAACCTCTACAAGCTCTTTACACTTT
TGGCCAGGGGACCAAGGTGGAGATCAAAACCACGACGCCAGCGCCGCGACCACCAAC
ACCGGCGCCCACCATCGCGTCGCAGCCCCTGTCCCTGCGCCCAGAGGCGTGCCGGCCA
GCGGCGGGGGGCGCAGTGCACACGAGGGGGCTGGACTTCGCCTGTGATATCTACATCTG
GGCGCCCTTGGCCGGGACTTGTGGGGTCCTTCTCCTGTCACTGGTTATCACCCTTTACTG
CTGTTGGCTTACAAAAAGAAGTATTCATCCAGTGTGCACGACCCTAACGGTGAATACA
TGTTCATGAGAGCAGTGAACACAGCCAAAAAATCTAGACTCACAGATGTGACCCTAAA
GCGCGGCCGCAAGAAGCTGCTGTACATCTTCAAGCAGCCCTTCATGCGCCCCGTGCAGA
CCACCCAGGAGGAGGACGGCTGtAGCTGCCGCTTCCCCGAGGAGGAGGAGGGCGGCTG
CGAGCTGCGCGTGAAATTTAGCCGCAGCGCCGACGCCCCCGCCTACCAGCAGGGCCAG
AACCAGCTGTACAACGAGCTGAACCTGGGCCGCCGCGAGGAGTACGACGTGCTGGACA
AGCGCCGGGGCCGCGACCCCGAGATGGGCGGCAAGCCCCAGCGCCGCAAGAACCCCC
AGGAGGGCCTGTACAACGAGCTGCAGAAGGACAAGATGGCCGAAGCCTACAGCGAGAT
CGGCATGAAGGGCGAGCGCCGCCGGGGCAAGGGCCACGACGGCCTGTACCAGGGCCT
GAGCACCGCCACCAAGGACACCTACGACGCCCTGCACATGCAGGCCCTGCCCCCCCGC
(SEQ ID NO: 117).

Secifically, the chimeric antigen receptor D72-CAR comprises the nucleotide sequence shown in SEQ ID NO:118.

CAGGTGCAGCTGGTGCAATCTGGGGCTGAGGTGAAGAAGCCTGGGTCCTCGGTGA
AGGTCTCCTGCAAGGCTTCTGGAGGCACCTTCAGCAGCTATGCTATCAGCTGGGTGCGA
CAGGCCCCTGGACAAGGGCTTGAGTGGATGGGAGGGATCATCCCTATCTTTGGTACAGC
AAACTACGCACAGAAGTTCCAGGGCAGAGTCACGATTACCGCGGACGAATCCACGAGC
ACAGCCTACATGGAGCTGAGCAGCCTGAGATCTGAGGACACGGCCGTGTATTACTGTGC

GAGAACGGGCGGTGATTACTACTACTACGGTATGGACGTCTGGGGCCAAGGGACCACG
GTCACCGTCTCCTCAGGTGGCGGCGGTTCAGGCGGAGGTGGCTCTGGCGGTGGCGGAT
CGGCCATCTGGATGACCCAGTCTCCATCCTCCCTGTCTGCATCTGTAGGAGACAGAGTCA
CCATCACTTGTCGGGCAAGTCAGACCATAAGCACCTATTTAAATTGGTATCAACAGAAAC
CAGGGAAAGCCCCTAAACTCCTGATCTATGGTGCATCCAGTTTGCAAACTGGGGTCCCA
TCAAGGTTCAGTGGCAGTGGATCTGGGACAGATTTCACTCTCACCATCAGCAGTCTGCA
ACCTGAAGATTTTGCAACTTACTACTGTCAACAGAGTTACAGTACCCCTCCCACTTTTGG
CCAGGGGACCAAAGTGGATATCAAAACCACGACGCCAGCGCCGCGACCACCAACACCG
GCGCCCACCATCGCGTCGCAGCCCCTGTCCCTGCGCCCAGAGGCGTGCCGGCCAGCGG
CGGGGGGCGCAGTGCACACGAGGGGGCTGGACTTCGCCTGTGATATCTACATCTGGGCG
CCCTTGGCCGGGACTTGTGGGGTCCTTCTCCTGTCACTGGTTATCACCCTTTACTGCTGT
TGGCTTACAAAAAGAAGTATTCATCCAGTGTGCACGACCCTAACGGTGAATACATGTT
CATGAGAGCAGTGAACACAGCCAAAAAATCTAGACTCACAGATGTGACCCTAAAGCGC
GGCCGCAAGAAGCTGCTGTACATCTTCAAGCAGCCCTTCATGCGCCCCGTGCAGACCAC
CCAGGAGGAGGACGGCTGTAGCTGCCGCTTCCCCGAGGAGGAGGAGGGCGGCTGCGA
GCTGCGCGTGAAATTTAGCCGCAGCGCCGACGCCCCCGCCTACCAGCAGGGCCAGAAC
CAGCTGTACAACGAGCTGAACCTGGGCCGCCGCGAGGAGTACGACGTGCTGGACAAGC
GCCGGGGCCGCGACCCCGAGATGGGCGGCAAGCCCCAGCGCCGCAAGAACCCCCAGG
AGGGCCTGTACAACGAGCTGCAGAAGGACAAGATGGCCGAAGCCTACAGCGAGATCGG
CATGAAGGGCGAGCGCCGCCGGGGCAAGGGCCACGACGGCCTGTACCAGGGCCTGAG
CACCGCCACCAAGGACACCTACGACGCCCTGCACATGCAGGCCCTGCCCCCCCGC (SEQ
ID NO: 118).

Secifically, the chimeric antigen receptor D76-CAR comprises the nucleotide sequence shown in SEQ ID NO:160.

ATGGGCGTCAAGGTCCTGTTCGCCCTGATCTGCATCGCCGTCGCCGAGGCCGAGGT
GCAGCTGGTGGAGTCTGGAGCTGAGGTGAAGAAGCCTGGGGCCTCAGTGAAGGTCTCC
TGCAAGGCTTCTGGATACACCTTCACCGACTACTTTCTACACTGGGTGCGACAGGCCCC
TGGACAAGGGCTTGAGTGGATGGGTTGGATCAACCCTAACAGCGGCGGCACAAACTAT
GCACAGAAGTTTCAGGGTTGGGTCACCATGACCAGGGACACGTCCATCAGCACAGCCT
ACTTGGAGCTGAGCAGCCTAACATCTGACGACACGGCCGTTTATTACTGTGCTGTGGAC
CCCTTTGACTACTGGGGCCAGGGAACCCTGGTCACCGTCTCCTCAGGTGGCGGCGGTTC
AGGCGGAGGTGGCTCTGGCGGTGGCGGATCGGATGTTGTGATGACTCAGTCTCCACTCT
CCTCACCTGTCACCCTTGGACAGCCGGCCTCCATCTCCTGCAGGTCTAGTCAAAGCCTC
GTACATAGTGATGGAAACACCTACTTGAGTTGGCTTCACCAGAGGCCAGGCCAGCCTCC
AAGACTCCTAATTCATAAGATTTCTAACCGGTTCTCTGGGGTCCCAGACAGATTCAGTGG
CAGTGGGGCAGGGACAGATTTCACACTGAAAATCAGCAGGGTGGAAGCTGAGGATGTC
GGGGTTTTTTACTGCATGCAAGCTACACAATTTCCGATCACCTTCGGCCAAGGGACACG

ACTGGAGATTAAAACCACGACGCCAGCGCCGCGACCACCAACACCGGCGCCCACCATC
GCGTCGCAGCCCCTGTCCCTGCGCCCAGAGGCGTGCCGGCCAGCGGCGGGGGGCGCAG
TGCACACGAGGGGGCTGGACTTCGCCTGTGATATCTACATCTGGGCGCCCTTGGCCGGG
ACTTGTGGGGTCCTTCTCCTGTCACTGGTTATCACCCTTTACTGCTGTTGGCTTACAAAA
AAGAAGTATTCATCCAGTGTGCACGACCCTAACGGTGAATACATGTTCATGAGAGCAGT
GAACACAGCCAAAAAATCTAGACTCACAGATGTGACCCTAAAGCGCGGCCGCAAGAAG
CTGCTGTACATCTTCAAGCAGCCCTTCATGCGCCCCGTGCAGACCACCCAGGAGGAGGA
CGGCTGtAGCTGCCGCTTCCCCGAGGAGGAGGAGGGCGGCTGCGAGCTGCGCGTGAAA
TTTAGCCGCAGCGCCGACGCCCCCGCCTACCAGCAGGGCCAGAACCAGCTGTACAACG
AGCTGAACCTGGGCCGCCGCGAGGAGTACGACGTGCTGGACAAGCGCCGGGGCCGCG
ACCCCGAGATGGGCGGCAAGCCCCAGCGCCGCAAGAACCCCCAGGAGGGCCTGTACAA
CGAGCTGCAGAAGGACAAGATGGCCGAAGCCTACAGCGAGATCGGCATGAAGGGCGA
GCGCCGCCGGGGCAAGGGCCACGACGGCCTGTACCAGGGCCTGAGCACCGCCACCAA
GGACACCTACGACGCCCTGCACATGCAGGCCCTGCCCCCCGC (SEQ ID NO: 160).

Secifically, the chimeric antigen receptor D79-CAR comprises the nucleotide sequence shown in SEQ ID NO:161.

ATGGGCGTCAAGGTCCTGTTCGCCCTGATCTGCATCGCCGTCGCCGAGGCCCAGGT
CCAGCTGGTGCAATCTGGGGCTGAGGTGAAGAAGCCTGGGGCCTCAGTGAAGGTCTCC
TGCAAGGCTTCTGGATACACCTTCACCGGCTACTATATGCACTGGGTGCGACAGGCCCCT
GGACAAGGGCTTGAGTGGATGGGATGGATCAACCCTAACAGTGGTGGCACAAACTATG
CACAGAAGTTTCAGGGCAGGGTCACCATGACCAGGGACACGTCCATCAGCACAGCCTA
CATGGAGCTGAGCAGGCTGAGATCTGACGACACGGCCGTGTATTACTGTGCGAGAGTTG
GTGGGAGCTATTACGGGGGGTTTGACTACTGGGGCCAGGGAACCCTGGTCACCGTCTCC
TCAGGTGGCGGCGGTTCAGGCGGAGGTGGCTCTGGCGGTGGCGGATCGGAAATTGTGC
TGACTCAGTCTCCCCTCTCTCTGCCCGTCACCCCTGGAGAGCCGGCCTCCATCTCCTGCA
GGTCTAGTCAGAGCCTCCTGCATAGTAATGGATACAACTATTTGGATTGGTACCTGCAGA
AGCCAGGACAGTCTCCACAGCTCCTGATGTATTTGAGTTCTAATCGGGCCTCCGGGGTC
CCTGACAGGTTCAGTGGCAGTGGATCAGGCAGAGATTTTACACTGAAAATCAGCAGAGT
GGAGGCTGAGGATGTTGGGCTTTATTATTGCATGCAAGCTCTACAAACTCCTCCCACCTT
CGGCCAAGGGACACGACTGGAGATTAAACCACGACGCCAGCGCCGCGACCACCAAC
ACCGGCGCCCACCATCGCGTCGCAGCCCCTGTCCCTGCGCCCAGAGGCGTGCCGGCCA
GCGGCGGGGGGCGCAGTGCACACGAGGGGGCTGGACTTCGCCTGTGATATCTACATCTG
GGCGCCCTTGGCCGGGACTTGTGGGGTCCTTCTCCTGTCACTGGTTATCACCCTTTACTG
CTGTTGGCTTACAAAAAAGAAGTATTCATCCAGTGTGCACGACCCTAACGGTGAATACA
TGTTCATGAGAGCAGTGAACACAGCCAAAAAATCTAGACTCACAGATGTGACCCTAAA
GCGCGGCCGCAAGAAGCTGCTGTACATCTTCAAGCAGCCCTTCATGCGCCCCGTGCAGA
CCACCCAGGAGGAGGACGGCTGTAGCTGCCGCTTCCCCGAGGAGGAGGAGGGCGGCT

GCGAGCTGCGCGTGAAATTTAGCCGCAGCGCCGACGCCCCCGCCTACCAGCAGGGCCA
GAACCAGCTGTACAACGAGCTGAACCTGGGCCGCCGCGAGGAGTACGACGTGCTGGAC
AAGCGCCGGGGCCGCGACCCCGAGATGGGCGGCAAGCCCAGCGCCGCAAGAACCCC
CAGGAGGGCCTGTACAACGAGCTGCAGAAGGACAAGATGGCCGAAGCCTACAGCGAG
ATCGGCATGAAGGGCGAGCGCCGCCGGGGCAAGGGCCACGACGGCCTGTACCAGGGCC
TGAGCACCGCCACCAAGGACACCTACGACGCCCTGCACATGCAGGCCCTGCCCCCCG
C (SEQ ID NO: 161).

Secifically, the chimeric antigen receptor D85-CAR comprises the nucleotide sequence shown in SEQ ID NO:162.

ATGGGCGTCAAGGTCCTGTTCGCCCTGATCTGCATCGCCGTCGCCGAGGCCCAGGT
GCAGCTGGTGCAATCTGGGGCTGAGGTGAAGAAGCCTGGGGCCTCAGTGAAGGTCTCC
TGCAAGGCTTCTGGATACACCTTCACCGGCTACTATATGCACTGGGTGCGACAGGCCCCT
GGACAAGGGCTTGAGTGGATGGGATGGATCAACCCTAACAGTGGTGGCACAAACTATG
CACAGAAGTTTCAGGGCAGGGTCACCATGACCAGGGACACGTCCATCAGCACAGCCTA
CATGGAGCTGAGCAGGCTGAGATCTGACGACACGGCCGTGTATTACTGTGCGAGTGACC
CCTTTGACTACTGGGGCCAGGGAACCCTGGTCACCGTCTCCTCAGGTGGCGGCGGTTCA
GGCGGAGGTGGCTCTGGCGGTGGCGGATCGGATGTTGTGATGACTCAGTCTCCACTCTC
CTCACCTGTCACCCTTGGACAGCCGGCCTCCATCTCCTGCAGGTCTAGTCAAAGCCTCG
TATACAGTGATGGGAACACCTACTTGAGTTGGCTTCAGCAGAGGCCAGGCCAGCCTCCA
AGACTCCTAATTTATAAGGTTTCTAACCGGTTCTCTGGGGTCCCAGACAGATTCAGTGGC
AGTGGGGCAGGGACAGATTTCACACTGACAATCAGCAGGGTGGAAGCTGAGGATGTCG
GAGTTTATTACTGCATGCAAGCTACACAATATCCGCTCGCTTTCGGCGGAGGGACCAAGC
TGGAGATCAAACCACGACGCCAGCGCCGCGACCACCAACACCGGCGCCCACCATCGC
GTCGCAGCCCCTGTCCCTGCGCCCAGAGGCGTGCCGGCCAGCGGCGGGGGGCGCAGTG
CACACGAGGGGGCTGGACTTCGCCTGTGATATCTACATCTGGGCGCCCTTGGCCGGGAC
TTGTGGGGTCCTTCTCCTGTCACTGGTTATCACCCTTTACTGCTGTTGGCTTACAAAAA
GAAGTATTCATCCAGTGTGCACGACCCTAACGGTGAATACATGTTCATGAGAGCAGTGA
ACACAGCCAAAAAATCTAGACTCACAGATGTGACCCTAAAGCGCGGCCGCAAGAAGCT
GCTGTACATCTTCAAGCAGCCCTTCATGCGCCCCGTGCAGACCACCCAGGAGGAGGACG
GCTGTAGCTGCCGCTTCCCCGAGGAGGAGGAGGGCGGCTGCGAGCTGCGCGTGAAATT
TAGCCGCAGCGCCGACGCCCCCGCCTACCAGCAGGGCCAGAACCAGCTGTACAACGAG
CTGAACCTGGGCCGCCGCGAGGAGTACGACGTGCTGGACAAGCGCCGGGGCCGCGACC
CCGAGATGGGCGGCAAGCCCCAGCGCCGCAAGAACCCCCAGGAGGGCCTGTACAACG
AGCTGCAGAAGGACAAGATGGCCGAAGCCTACAGCGAGATCGGCATGAAGGGCGAGC
GCCGCCGGGGCAAGGGCCACGACGGCCTGTACCAGGGCCTGAGCACCGCCACCAAGG
ACACCTACGACGCCCTGCACATGCAGGCCCTGCCCCCCCGC (SEQ ID NO: 162).

Secifically, the chimeric antigen receptor D103-CAR comprises the nucleotide sequence shown in SEQ ID NO:163.

ATGGGCGTCAAGGTCCTGTTCGCCCTGATCTGCATCGCCGTCGCCGAGGCCCAGGT
GCAGCTGGTGCAATCTGGAGCAGAGGTGAAAAAGCCCGGGGAGTCTCTGAAGATCTCC
TGTAAGGGTTCTGGATACAGCTTTACCAACTACTGGATCGGCTGGGTGCGCCAGATGCC
CGGGAAAGGCCTGGAGTGGATGGGGATCATCTATCCTGGTGACTCTGATACCAGATACA
GCCCGTCCTTCCAAGGCCAGGTCACCATCTCAGCCGACAAGTCCATCAGCACCGCCTAC
CTGCAGTGGAGCAGCCTGAAGGCCTCGGACACCGCCATGTATTACTGTGCGAGACCCCA
GGGAGCTTTTGATATCTGGGGCCAAGGGACAATGGTCACCGTCTCCTCAGGTGGCGGCG
GTTCAGGCGGAGGTGGCTCTGGCGGTGGCGGATCGGAGATTGTGATGACCCAGACTCC
ACTCTCCCTGCCCGTCACCCTTGGACAGCCGGCCTCCATCTCCTGCAGGTCTAGTCAAA
GCCTCGTATACAGTGATGGAAACACCTACTTGAATTGGTTTCAGCAGAGGCCAGGCCAA
TCTCCAAGGCGCCTAATTTATAAGGTTTCTAACCGGGACTCTGGGGTCCCAGACAGATTC
AGCGGCAGTGGGTCAGGCACTGATTTCACACTGAAAATCAGCAGGGTGGAGGCTGAGG
ATGTTGGGGTTTATTACTGCATGCAAGGTACACACTGGCCTCTGACGTTCGGCCAAGGG
ACCAAGGTGGAGATCAAAACCACGACGCCAGCGCCGCGACCACCAACACCGGCGCCC
ACCATCGCGTCGCAGCCCCTGTCCCTGCGCCCAGAGGCGTGCCGGCCAGCGGCGGGG
GCGCAGTGCACACGAGGGGGCTGGACTTCGCCTGTGATATCTACATCTGGGCGCCCTTG
GCCGGGACTTGTGGGGTCCTTCTCCTGTCACTGGTTATCACCCTTTACTGCTGTTGGCTT
ACAAAAAGAAGTATTCATCCAGTGTGCACGACCCTAACGGTGAATACATGTTCATGAG
AGCAGTGAACACAGCCAAAAAATCTAGACTCACAGATGTGACCCTAAAGCGCGGCCGC
AAGAAGCTGCTGTACATCTTCAAGCAGCCCTTCATGCGCCCCGTGCAGACCACCCAGGA
GGAGGACGGCTGTAGCTGCCGCTTCCCCGAGGAGGAGGAGGGCGGCTGCGAGCTGCGC
GTGAAATTTAGCCGCAGCGCCGACGCCCCCGCCTACCAGCAGGGCCAGAACCAGCTGT
ACAACGAGCTGAACCTGGGCCGCCGCGAGGAGTACGACGTGCTGGACAAGCGCCGGG
GCCGCGACCCCGAGATGGGCGGCAAGCCCAGCGCCGCAAGAACCCCCAGGAGGGCC
TGTACAACGAGCTGCAGAAGGACAAGATGGCCGAAGCCTACAGCGAGATCGGCATGAA
GGGCGAGCGCCGCCGGGGCAAGGGCCACGACGGCCTGTACCAGGGCCTGAGCACCGC
CACCAAGGACACCTACGACGCCCTGCACATGCAGGCCCTGCCCCCCCGC (SEQ ID NO:
163).

In some embodiments, the nucleotide sequence further comprises a restriction enzyme cleavage site.

Specifically, the restriction enzyme cleavage site is selected from BamHI, SpeI, SalI, KpnI, XhoI, SphI, XbaI, EcoRI, and the like.

In some embodiments, the nucleotide sequence of the EcoRI restriction enzyme cleavage site is: GAATTC.

As a specific example, the chimeric antigen receptor D9-CAR comprising a restriction enzyme cleavage site comprises the nucleotide sequence shown in SEQ ID NO:126:
GAGGTGCATCTGGTGGAGTCTGGGGGAGGCGTGGTCCAGCCTGGGAGGTCCCTGA GACTCTCCTGTGCAGCCTCTGGATTCACCTTCAGTAACTATGCTATGCACTGGGTCCGCC AGGCTCCAGGCAAGGGGCTGGAGTGGGTGGCAGTTATCTCATATGATGGAAGTAAAAAA TACTACGCAGACTCCGTGAAGGGCCGATTCACCATCTCCAGAGACGATTCCAAGAACAC GCTGTATCTGCAAATGAACAGCCTGAGAGCCGAGGACACGGCCGTATATTACTGTGCGA AACCCGCTGGTAGTACCAGTGGGTTTGACTACTGGGGCCAGGGAACCCTGGTCACCGTC TCCTCAGGTGGCGGCGGTTCAGGCGGAGGTGGCTCTGGCGGTGGCGGATCGCAGTCTG CCCTGACTCAGCCTGCCTCCGTGTCTGGGTCTCCTGGACAGTCGATCACCATCTCCTGCA CTGGAACCAGCAGTGACGTTGGTGGTTATAACTATGTCTCCTGGTACCAACAACACCCA GGCAAAGCCCCCAAACTCATGATTTATGATGTCAGTAATCGGCCCTCAGGGGTTTCTAAT CGCTTCTCTGGCTCCAAGTCTGGCAACACGGCCTCCCTGACCATCTCTGGGCTCCAGGC TGAGGACGAGGCTGATTATTACTGCAGCTCATATACAAGCAGCAGCACTCTCGTGGTATT CGGCGGAGGGACCAAGCTGACCGTCCTA**GAATTC**ACCACGACGCCAGCGCCGCGACC ACCAACACCGGCGCCCACCATCGCGTCGCAGCCCCTGTCCCTGCGCCCAGAGGCGTGC CGGCCAGCGGCGGGGGGCGCAGTGCACACGAGGGGGCTGGACTTCGCCTGTGATATCT ACATCTGGGCGCCCTTGGCCGGGACTTGTGGGGTCCTTCTCCTGTCACTGGTTATCACCC TTTACTGCTGTTGGCTTACAAAAAAGAAGTATTCATCCAGTGTGCACGACCCTAACGGT GAATACATGTTCATGAGAGCAGTGAACACAGCCAAAAAATCTAGACTCACAGATGTGAC CCTAAAGCGCGGCCGCAAGAAGCTGCTGTACATCTTCAAGCAGCCCTTCATGCGCCCCG TGCAGACCACCCAGGAGGAGGACGGCTGTAGCTGCCGCTTCCCCGAGGAGGAGGAGG GCGGCTGCGAGCTGCGCGTGAAATTTAGCCGCAGCGCCGACGCCCCCGCCTACCAGCA GGGCCAGAACCAGCTGTACAACGAGCTGAACCTGGGCCGCCGCGAGGAGTACGACGTG CTGGACAAGCGCCGGGGCCGCGACCCCGAGATGGGCGGCAAGCCCCAGCGCCGCAAG AACCCCCAGGAGGGCCTGTACAACGAGCTGCAGAAGGACAAGATGGCCGAAGCCTACA GCGAGATCGGCATGAAGGGCGAGCGCCGCCGGGGCAAGGGCCACGACGGCCTGTACCA GGGCCTGAGCACCGCCACCAAGGACACCTACGACGCCCTGCACATGCAGGCCCTGCCC CCCCGC (SEQ ID NO: 126), wherein the bolded portion represents the nucleotide sequence of the EcoRI restriction enzyme cleavage site;

The chimeric antigen receptors described in the present invention may all comprise a nucleotide sequence containing a restriction enzyme site, as illustrated in the specific examples above, wherein the restriction enzyme site is located upstream of the nucleotide sequence of the hinge region and downstream of the nucleotide sequence of the extracellular antigen-binding structure.

In yet another aspect, the present invention provides a nucleic acid construct comprising the nucleic acid molecule described herein; further, the nucleic acid construct comprises one or more control sequences operably linked to the nucleic acid molecule, wherein the one or more control sequences direct the production of the antibody or antigen-binding fragment thereof described herein in a suitable host cell, and the one or more control sequences are selected from the group consisting of: a promoter, an enhancer, a stop signal, a signal peptide, a leader sequence, a transcription terminator, and any combination thereof.

In some embodiments, the signal peptide described in the present invention has the nucleotide sequence set forth in SEQ ID NO:119.
ATGGGCGTCAAGGTCCTGTTCGCCCTGATCTGCATCGCCGTCGCCGAGGCC (SEQ ID NO: 119).

In yet another aspect, provided herein is an expression vector carrying the nucleic acid molecule of the present invention.

In some embodiments, the expression vector is a γ-retroviral vector or a lentiviral vector.

In yet another aspect, the present invention provides an immune effector cell comprising the chimeric antigen receptor described herein, the nucleic acid molecule described herein, or the expression vector described herein.

In some embodiments, the immune effector cell is selected from T cells, natural killer cells, cytotoxic T lymphocytes, regulatory T cells, human embryonic stem cells, lymphoid progenitor cells, T cell precursor cells, or pluripotent stem cells capable of differentiating into lymphocytes, macrophages, B cells, and immune cells derived from induced pluripotent stem cells that have cell-killing capability.

In yet another aspect, the present invention provides a method for preparing an immune effector cell, the method comprising introducing the expression vector described herein into an immune effector cell.

In yet another aspect, the present invention provides a composition comprising the immune effector cell described herein and a pharmaceutically acceptable carrier.

In yet another aspect, the present invention provides the use of the chimeric antigen receptor of the present invention, the nucleic acid molecule of the present invention, the expression vector of the present invention, the immune effector cell of the present invention, or the composition of the present invention in the manufacture of a medicament, wherein the medicament is used for treating a disease or condition associated with the expression of Ly6G6D; wherein the disease or condition associated with the expression of Ly6G6D is colorectal cancer, esophageal cancer, gastric cancer, small intestine cancer, large intestine cancer, or adenocarcinoma (e.g., colorectal adenocarcinoma, gastric adenocarcinoma, or pancreatic adenocarcinoma), or metastatic cancer thereof, such as metastatic colorectal adenocarcinoma.

In yet another aspect, the present invention provides a kit for treating cancer, wherein the kit comprises the immune effector cell of the present invention.

In some embodiments, the kit of the present invention further comprises instructions for using the immune effector cell to treat a subject with cancer.

In some embodiments, the cancer of the present invention is colorectal cancer, esophageal cancer, gastric cancer, small intestine cancer, large intestine cancer, or adenocarcinoma (e.g., colorectal adenocarcinoma, gastric adenocarcinoma, or pancreatic adenocarcinoma) , or metastatic cancer thereof, such as metastatic colorectal adenocarcinoma.

### Definitions and General Terms

In the present invention, unless otherwise specified, scientific and technical terms used herein shall have the meanings commonly understood by those skilled in the art. Furthermore, the operational steps for cell culture, molecular biology, biochemistry, nucleic acid chemistry, immunology, and the like used herein are conventional steps widely used in the respective fields. Meanwhile, to better understand the present invention, definitions and explanations of relevant terms are provided below.

When the terms "for example," "such as," "including," "comprising," or variants thereof are used herein, these terms shall not be considered limiting but shall be interpreted to mean "but not limited to" or "not limited to."

Unless otherwise indicated herein or clearly contradicted by context, the terms "a", "an", "the" and similar referents in the context of describing the present invention (especially in the context of the following claims) shall be construed to cover both the singular and the plural.

### Chimeric Antigen Receptor

The term "Chimeric Antigen Receptor" (CAR) refers to an engineered receptor that grafts or confers specificity of interest onto an immune effector cell. CAR can be used to graft the specificity of a monoclonal antibody onto T cells by facilitating the transfer of its coding sequence via a retroviral vector.

CARs are divided into three generations "First-generation" CARs typically consist of an extracellular antigen-binding domain (e.g., a single-chain variable fragment (scFv)) fused to a transmembrane domain, which is further fused to the cytoplasmic/intracellular domain of a T cell receptor chain. "First-generation" CARs usually contain an intracellular domain derived from the CD3ζ chain, which is the primary transmitter of signaling from the endogenous TCR. First-generation CARs can provide de novo antigen recognition and, through their CD3ζ chain signaling domain in a single fusion molecule, lead to the activation of CD4+ and CD8+ T cells, independent of HLA-mediated antigen presentation. "Second-generation" CARs add intracellular domains from various costimulatory molecules (e.g., CD28, 4-1BB, ICOS, OX40) to the cytoplasmic region of the CAR to provide additional signals to T cells. "Second-generation" CARs include those that provide both costimulation (e.g., CD28 or 4-1BB) and activation (CD3ζ). Preclinical studies have shown that second-generation CARs can enhance the antitumor activity of T cells. For example, in patients with chronic lymphocytic leukemia (CLL) and acute lymphoblastic leukemia (ALL), clinical trials targeting the CD19 molecule have demonstrated strong efficacy of second-generation CAR-modified T cells. "Third-generation" CARs include those that provide multiple costimulatory signals (e.g., CD28 and 4-1BB) and activation (CD3ζ).

According to the subject matter disclosed herein, the CAR comprises an extracellular antigen-binding domain, a transmembrane domain, and an intracellular domain, wherein the extracellular antigen-binding domain binds to Ly6G6D. In certain embodiments, the extracellular antigen-binding domain is an scFv. In certain embodiments, the extracellular antigen-binding domain is an optionally cross-linked Fab. In certain embodiments, the extracellular binding domain is an F(ab)2. In certain embodiments, any of the foregoing molecules may be included in a fusion protein containing a heterologous sequence to form the extracellular antigen-binding domain. In one specific, non-limiting embodiment, the extracellular antigen-binding domain comprises a humanized scFv that specifically binds to human Ly6G6D.

In certain non-limiting embodiments, the extracellular antigen-binding domain of the CAR exhibits high binding specificity and high binding affinity for Ly6G6D. For example, in these embodiments, the extracellular antigen-binding domain of the CAR (e.g., implemented as a humanized scFv or an analog thereof) binds to Ly6G6D with a dissociation constant (KD) of about 1 x 10⁻⁹ M or lower.

The term "Transmembrane Domain" generally refers to the domain in a CAR that traverses the cell membrane, which is connected to the intracellular signal transduction domain and functions to transmit signals.

The term "costimulatory domain" generally refers to an intracellular domain capable of providing an immune co-stimulatory molecule, wherein said co-stimulatory molecule is a cell surface molecule required for an effective response of lymphocytes to an antigen. The costimulatory domain may include costimulatory domains of members of the immunoglobulin superfamily (IgSF), such as CD28 and ICOS, and may also include costimulatory domains of members of the tumor necrosis factor receptor superfamily (TNFRSF), such as CD27, OX40, and 4-1BB.

CDR domains are defined using the Kabat system (Kabat, E. A. et al. (1991) Sequences of Proteins of Immunological Interest, 5th Ed., U.S. Department of Health and Human Services, NIH Publication No. 91-3242). The subject matter disclosed herein further provides conservatively modified extracellular antigen-binding domains (e.g., scFv) comprising the antibody sequences disclosed herein. For example, without limitation, the extracellular antigen-binding domains (e.g., scFv) of the subject matter disclosed herein comprise: a heavy chain variable region comprising CDR1, CDR2, and CDR3 sequences, and a light chain variable region comprising CDR1, CDR2, and CDR3 sequences, wherein one or more of these CDR sequences comprise a specific amino acid sequence disclosed herein or a conservative modification thereof, wherein the extracellular antigen-binding domain retains the desired functional properties.

The term "antibody" generally refers to a polypeptide molecule capable of specifically recognizing and/or neutralizing a specific antigen. For example, an antibody may comprise an immunoglobulin consisting of at least two heavy (H) chains and two light (L) chains interconnected by disulfide bonds, and includes any molecule containing its antigen-binding domain. "Antibody" includes monoclonal antibody, antibody fragment, or antibody derivative, including but not limited to human antibody, humanized antibody, chimeric antibody, single-domain antibody (e.g., dAb), single-chain antibody (e.g., scFv), and antibody fragment that bind to antigen (e.g., Fab, Fab', and (Fab)2 fragment). "Antibody" also includes all recombinant forms of antibodies, such as antibodies expressed in prokaryotic cells, non-glycosylated antibodies, and any antigen-binding antibody fragments and derivatives thereof described in this application. Each heavy chain may consist of a heavy chain variable region (VH) and a heavy chain constant region. Each light chain may consist of a light chain variable region (VL) and a light chain constant region. The VH and VL regions can be further subdivided into hypervariable regions called complementarity-determining regions (CDRs), which are interspersed within more conserved regions called framework regions (FRs). Each VH and VL may consist of three CDRs and four FRs, arranged from the amino terminus to the carboxyl terminus in the following order: FR1, CDR1, FR2, CDR2, FR3, CDR3, and FR4. The variable regions of the heavy and light chains contain binding domains that interact with the antigen. The constant regions of the antibody may mediate the binding of the immunoglobulin to host tissues or factors, said host tissues or factors including various cells of the immune system (e.g., effector cells) and the first component of the classical complement system (C1q).

The terms "binding domain," "extracellular domain," "extracellular antigen-binding domain," "extracellular binding domain," "antigen-specific binding domain," and "extracellular antigen-specific binding domain" are used interchangeably and refer to a domain or fragment of a CAR that provides the ability to specifically bind to a target antigen of interest (e.g., Ly6G6D). Examples include antibodies and their antigen-binding fragments, single-chain scFv antibodies, and various fusion proteins and conjugates constructed based on scFv, such as scFv-Fc antibodies, immunoconjugates, antibody-drug conjugates (ADCs), multi-/bispecific antibodies, and chimeric antigen receptors (CARs).

The term"single-chain variable fragment"(scFv) may refer to an antibody formed by linking said heavy chain variable region and said light chain variable region via a linker peptide.

In certain embodiments, no more than one, no more than two, no more than three, no more than four, or no more than five residues are altered within a specific sequence or CDR region. Exemplary conservative amino acid substitutions are shown in Table 1.

**Table 1**

| Original Residue | Exemplary Conservative Amino Acid Substitutions |
|---|---|
| Ala(A) | Val; Leu; Ile |
| Arg (R) | Lys; Gln; Asn |
| Asn (N) | Gln; His; Asp, Lys; Arg |
| Asp (D) | Glu; Asn |
| Cys(C) | Ser; Ala |
| Gln (Q) | Asn; Glu |
| Glu (E) | Asp; Gln |
| Gly (G) | Ala |
| His (H) | Asn; Gln; Lys; Arg |
| Ile (I) | Leu; Val; Met; Ala; Phe |
| Leu (L) | Ile;Val; Met; Ala; Phe |
| Lys (K) | Arg; Gln;Asn |
| Met (M) | Leu; Phe; Ile |
| Phe (F) | Trp; Leu; Val; Ile; Ala; Tyr |
| Pro (P) | Ala |
| Ser (S) | Thr |
| Thr (T) | Val; Ser |
| Trp (W) | Tyr; Phe |
| Tyr (Y) | Trp; Phe; Thr; Ser |
| Val (V) | Ile; Leu; Met; Phe; Ala |

The terms "conservative sequence modification" and "conservative sequence substitution" are used interchangeably and refer to amino acid modifications that do not significantly affect or alter the binding characteristics of the disclosed CARs of the present invention (e.g., extracellular antigen-binding domains). Such conservative modifications include amino acid substitutions, additions, and deletions. Modifications can be introduced into the disclosed scFvs of the present invention using standard techniques known in the art, such as site-directed mutagenesis and PCR-mediated mutagenesis. Amino acids can be grouped based on their physicochemical properties, such as charge and polarity. Conservative amino acid substitutions are those in which an amino acid residue is replaced by an amino acid within the same group. For example, amino acids can be classified by charge: positively charged amino acids include lysine, arginine, and histidine; negatively charged amino acids include aspartic acid and glutamic acid; and neutral amino acids include alanine, asparagine, cysteine, glutamine, glycine, isoleucine, leucine, methionine, phenylalanine, proline, serine, threonine, tryptophan, tyrosine, and valine. Furthermore, amino acids can be classified by polarity: polar amino acids include arginine (basic polar), asparagine, aspartic acid (acidic polar), glutamic acid (acidic polar), glutamine, histidine (basic polar), lysine (basic polar), serine, threonine, and tyrosine; non-polar amino acids include alanine, cysteine, glycine, isoleucine, leucine, methionine, phenylalanine, proline, tryptophan, and valine. Accordingly, one or more amino acid residues within a CDR region can be replaced by other amino acid residues from the same group, and functional assays described herein can be used to assess the retained function of the modified antibody.

The terms "identity" and "sequence identity" are used interchangeably to refer to the matching of sequences between two polypeptides or between two nucleic acids. When a specific position in both sequences being compared is occupied by the same base or amino acid monomeric subunit (for example, when a specific position in each of two DNA molecules is occupied by adenine, or a specific position in each of two polypeptides is occupied by lysine), then the molecules are identical at that position. The "percent identity" between two sequences is a function of the number of matching positions shared by the two sequences divided by the number of positions compared, multiplied by 100. For example, if 6 out of 10 positions match in two sequences, then these two sequences have 60% identity.

In certain non-limiting embodiments, the extracellular antigen-binding domain of a CAR may include a linker connecting the heavy chain variable region and the light chain variable region of the extracellular antigen-binding domain. As used herein, the term "Linker" refers to a functional group (e.g., chemical or polypeptide) that covalently connects two or more polypeptides or nucleic acids such that they are linked to each other. As used herein, "peptide linker" refers to one or more amino acids used to couple two proteins together (e.g., coupling VH and VL domains). Non-limiting examples of peptide linkers are disclosed in Shen et al., Anal. Chem. 80(6):1910-1917 (2008) and WO2014/087010.

Furthermore, the extracellular antigen-binding domain may include a leader sequence or signal peptide that directs nascent proteins into the endoplasmic reticulum. A signal peptide or leader sequence may be required if the CAR is to be glycosylated and anchored in the cell membrane. A signal sequence or leader sequence may be a peptide sequence (approximately 4-30 amino acids in length) present at the N-terminus of a newly synthesized protein that guides the newly synthesized protein into the secretory pathway.

In certain non-limiting embodiments, the transmembrane domain of a CAR includes a hydrophobic alpha helix spanning at least a portion of the membrane. Different transmembrane domains result in different receptor stabilities. Following antigen recognition, receptors cluster and signals are transmitted to the cell. According to the subject matter disclosed herein, the transmembrane domain of a CAR may include a CD8 polypeptide, a CD28 polypeptide, a CD3ζ polypeptide, a CD4 polypeptide, a 4-1BB polypeptide, an OX40 polypeptide, an ICOS polypeptide, a CTLA-4 polypeptide, a PD-1 polypeptide, a LAG-3 polypeptide, a 2B4 polypeptide, a BTLA polypeptide, a synthetic peptide (not based on proteins involved in immune responses), or combinations thereof.

In certain non-limiting embodiments, the intracellular domain of the CAR can include a CD3ζ polypeptide capable of activating or stimulating cells (e.g., lymphoid lineage cells such as T cells). CD3ζ includes 3 ITAMs and transmits an activation signal to the cell (e.g., lymphoid lineage cells such as T cells) upon antigen binding.

In certain non-limiting embodiments, the intracellular domain of the CAR further includes at least one signaling region. The at least one signaling region can include a CD28 polypeptide, a 4-1BB polypeptide, an OX40 polypeptide, an ICOS polypeptide, a DAP-10 polypeptide, a PD-1 polypeptide, a CTLA-4 polypeptide, a LAG-3 polypeptide, a 2B4 polypeptide, a BTLA polypeptide, a synthetic peptide (not based on proteins involved in immune responses), or combinations thereof.

In certain embodiments, the signaling region is a costimulatory signaling region. In certain embodiments, the costimulatory region includes at least one costimulatory molecule that can provide optimal lymphocyte activation. As used herein, "co-stimulatory molecule" refers to a cell surface molecule other than an antigen receptor or its ligand that is required for an effective response of a lymphocyte to an antigen. The at least one co-stimulatory signaling region can include a CD28 polypeptide, a 4-1BB polypeptide, an OX40 polypeptide, an ICOS polypeptide, a DAP-10 polypeptide, or combinations thereof. A costimulatory molecule can bind to a costimulatory ligand, which is a protein expressed on the cell surface that, upon binding to its receptor, generates a costimulatory response, defined herein as an intracellular response that modulates stimulation when an antigen binds to its CAR molecule. Costimulatory ligands include, but are not limited to, CD80, CD86, CD70, OX40L, 4-1BBL, CD48, TNFRSF14, and PD-L1. As an example, the 4-1BB ligand (i.e., 4-1BBL) can bind to 4-1BB (also referred to as "CD137") to provide an intracellular signal that, in combination with CAR signaling, induces the effector cell function of CAR+ T cells.

In certain embodiments, the CAR includes: an extracellular antigen-binding region comprising a human scFv that specifically binds to human Ly6G6D; a transmembrane domain comprising a CD8 polypeptide; and an intracellular domain comprising a CD3ζ polypeptide and a costimulatory signaling region comprising a combination of 4-1BB and ICOS. In certain embodiments, the CAR further includes a signal peptide or leader sequence covalently linked to the 5' end of the extracellular antigen-binding domain.

In certain embodiments, the CAR of the disclosed subject matter can further include an inducible promoter for expressing a nucleic acid sequence in human cells. The promoter used to express the CAR gene can be a constitutive promoter.

### Immune effector cell

The subject matter disclosed herein provides immune effector cells expressing a CAR comprising an extracellular antigen-binding domain, a transmembrane domain, and an intracellular domain, as described above, wherein the extracellular antigen-binding domain specifically binds to Ly6G6D (e.g., human Ly6G6D). The immune effector cells can be transduced with the CAR disclosed herein such that the cells express the CAR. The subject matter disclosed herein further provides methods of treating tumors, such as rectal cancer, using such cells. The immune effector cells of the subject matter disclosed herein can be cells of the lymphoid lineage, including B cells, T cells, and natural killer (NK) cells of the lymphoid lineage, providing antibody production, regulation of the cellular immune system, detection of foreign substances in the blood, detection of host alien cells, etc. Non-limiting examples of cells of the lymphoid lineage include T cells, natural killer (NK) cells, cytotoxic T lymphocytes (CTLs), regulatory T cells, embryonic stem cells, and pluripotent stem cells (e.g., pluripotent stem cells capable of differentiating into lymphoid cells). T cells can be lymphocytes that mature in the thymus and are primarily responsible for cell-mediated immunity. T cells are involved in the adaptive immune system. The T cells of the subject matter disclosed herein can be any type of T cell, including but not limited to helper T cells, cytotoxic T cells, memory T cells (including central memory T cells, stem cell-like memory T cells (or stem-like memory T cells), and two types of effector memory T cells (e.g., TEM cells and TEMRA cells)), regulatory T cells (also known as suppressor T cells), natural killer T cells, mucosal-associated invariant T cells, and γδ T cells. In certain embodiments, T cells expressing a CAR express Foxp3 to achieve and maintain a T regulatory phenotype. Natural killer (NK) cells can be lymphocytes that function as part of cell-mediated immunity and play a role during innate immune responses. NK cells do not require prior activation to exert their cytotoxic effects on target cells. Cytotoxic T cells (CTLs or killer T cells) are a subtype of T lymphocytes capable of inducing death in infected somatic cells or tumor cells.

### Vector

Genetic modification of immune effector cells (e.g., T cells, CTL cells, NK cells) can be achieved by transducing a substantially homogeneous cell composition with recombinant DNA or RNA constructs. The vector can be a retroviral vector (e.g., gamma retrovirus) used to introduce the DNA or RNA construct into the host cell genome. For example, a polynucleotide encoding a CAR targeting Ly6G6D can be cloned into a retroviral vector, and expression can be driven by its endogenous promoter, a retroviral long terminal repeat, or an alternative internal promoter.

Non-viral vectors or RNA can also be used. Random chromosomal integration or targeted integration (e.g., using nucleases, transcription activator-like effector nucleases (TALENs), zinc finger nucleases (ZFNs), and/or clustered regularly interspaced short palindromic repeats (CRISPR)) or transgene expression (e.g., using native or chemically modified RNA) can be employed.

### Administration

The disclosed subject matter's CARs targeting Ly6G6D and immune effector cells expressing them can be provided systemically or directly to a subject for the treatment or prevention of cancer. In certain embodiments, the CARs targeting Ly6G6D and the immune effector cells expressing them are injected directly into the organ of interest. Alternatively, or additionally, the CARs targeting Ly6G6D and the immune effector cells expressing them are provided indirectly to the organ of interest, for example, by administration into the circulatory system (e.g., the tumor vasculature). Expansion agents and differentiation agents can be provided before, during, or after the administration of cells and compositions to increase T cell production *in vitro* or *in vivo*.

The compositions disclosed herein include pharmaceutical compositions comprising immune effector cells expressing a CAR targeting Ly6G6D and a pharmaceutically acceptable carrier. Administration may be autologous or allogeneic. For example, immune effector cells expressing a CAR targeting Ly6G6D and compositions comprising the same may be obtained from a subject and administered to the same subject or a different compatible subject. T cells derived from peripheral blood disclosed herein or progeny thereof (e.g., obtained in vivo, ex vivo followed by in vivo, or in vitro) may be administered via local injection, including catheter administration, systemic injection, local injection, intravenous injection, or parenteral administration. When administering the pharmaceutical compositions disclosed herein (e.g., pharmaceutical compositions comprising immune effector cells expressing a CAR targeting Ly6G6D), they may be formulated in unit dose injectable forms (solutions, suspensions, emulsions).

The term "pharmaceutically acceptable carrier" refers to a carrier that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), and includes but is not limited to: pH adjusters, surfactants, adjuvants, ionic strength enhancers, diluents, agents for maintaining osmotic pressure, agents for delaying absorption, and preservatives. For example, pH adjusters include but are not limited to phosphate buffers. Surfactants include but are not limited to cationic, anionic, or nonionic surfactants, such as Tween-80. Ionic strength enhancers include but are not limited to sodium chloride. Preservatives include but are not limited to various antibacterial and antifungal agents, such as parabens, chlorobutanol, phenol, sorbic acid, etc. Agents for maintaining osmotic pressure include but are not limited to sugars, NaCl, and analogs thereof. Agents for delaying absorption include but are not limited to monostearates and gelatin. Diluents include but are not limited to water, aqueous buffers (such as buffered saline), alcohols, and polyols (such as glycerin), etc. Preservatives include but are not limited to various antibacterial and antifungal agents, such as thimerosal, 2-phenoxyethanol, parabens, chlorobutanol, phenol, sorbic acid, etc. Stabilizers have the meaning commonly understood by those skilled in the art and are capable of stabilizing the desired activity of the active ingredient in the drug, including but not limited to monosodium glutamate, gelatin, SPGA, sugars (such as sorbitol, mannitol, starch, sucrose, lactose, dextran, or glucose), amino acids (such as glutamic acid, glycine), proteins (such as dried whey, albumin, or casein), or degradation products thereof (such as lactalbumin hydrolysate), etc. In certain exemplary embodiments, the pharmaceutically acceptable carrier includes sterile injectable liquids (such as aqueous or nonaqueous suspensions or solutions). In some exemplary embodiments, such sterile injectable liquids are selected from Water for Injection (WFI), Bacteriostatic Water for Injection (BWFI), sodium chloride solution (e.g., 0.9% (w/v) NaCl), glucose solution (e.g., 5% glucose), solutions containing surfactants (e.g., 0.01% polysorbate 20), pH buffer solutions (e.g., phosphate buffer solution), Ringer's solution, and any combination thereof.

### Therapeutic Methods

Tumor microenvironment. Tumors possess a microenvironment hostile to host immune responses, involving a series of mechanisms by malignant cells to protect themselves from immune recognition and elimination. This "hostile tumor microenvironment" includes various immunosuppressive factors, including infiltrating regulatory CD4+ T cells (Treg), myeloid-derived suppressor cells (MDSC), tumor-associated macrophages (TAM), immunosuppressive cytokines including IL-10 and TGF-β, as well as the expression of ligands targeting immunosuppressive receptors expressed by activated T cells (CTLA-4 and PD-1). These immunosuppressive mechanisms play a role in maintaining tolerance and suppressing inappropriate immune responses; however, within the tumor microenvironment, these mechanisms prevent effective anti-tumor immune responses. In summary, upon encountering targeted tumor cells, these immunosuppressive factors can induce significant inactivation or apoptosis of adoptively transferred CAR-modified T cells.

Challenges in tumor immunology. Effective tumor immunity requires tumor antigen recognition and unopposed tumor elimination by immune effector cells. Tumor antigens must contain peptide epitopes presented by the tumor and recognizable by specific cytotoxic T lymphocytes (CTL). Primed CTL must expand to sufficient numbers and migrate to the tumor site, where they mature into effectors to exert their functions, a process enhanced by helper T cells and suppressed by Treg and inhibitory macrophages.

Targeted T-cell therapy with engineered T lymphocytes. T-cell engineering is a groundbreaking strategy with the potential to address many shortcomings observed in earlier immunotherapy approaches.

Principles of genetic approaches: Cell engineering can be used to redirect T cells to tumor antigens and enhance T-cell function. One driving force behind genetic T-cell modification is the potential to enhance T-cell survival and expansion while counteracting T-cell death, anergy, and immunosuppression. Genetic targeting of T cells can also be improved to prevent undesired destruction of normal tissues.

Chimeric Antigen Receptor (CAR): By transferring genes encoding CARs, tumor-specific T cells can be generated. Second-generation CARs comprise a tumor antigen-binding domain fused to an intracellular signal transduction domain capable of activating T cells, along with costimulatory domains designed to enhance T cell potency and persistence. Thus, CAR design can coordinate antigen recognition and signal transduction, two functions physiologically mediated by two separate complexes: the TCR heterodimer and the CD3 complex. The extracellular antigen-binding domain of CARs is typically derived from murine monoclonal antibodies (mAbs), human monoclonal antibodies, camelid antibodies, or from receptors or their ligands. Consequently, antigen recognition is non-MHC-restricted, enabling the same CAR to be applicable to any patient expressing the target antigen. Antigen binding via the CAR triggers phosphorylation of Immunoreceptor Tyrosine-based Activation Motifs (ITAMs) within the intracellular domain, initiating the signal transduction cascade required for cytolysis induction, cytokine secretion, and proliferation. Since MHC restriction of antigen recognition is bypassed, the function of CAR-targeted T cells is not affected by HLA downregulation or defects in antigen processing mechanisms.

Requirements for T cell expansion and survival: Proliferation of tumor-specific T cells may be required both *in vivo* and *ex vivo*. T cell proliferation must be accompanied by T cell survival to allow absolute T cell expansion and persistence. To proliferate in response to antigen, T cells generally require receipt of two signals. One is provided by TCR recognition of antigen peptide/MHC complexes displayed on the surface of antigen-presenting cells (APCs). The other is provided by T cell costimulatory receptors, such as CD28 or 4-1BB receptors. Although the cytolytic activity of T cells generally does not require concomitant costimulation, as previously demonstrated, there is a critical need to provide costimulatory signals to maintain the antitumor function of adoptively transferred T cells.

Immune Monitoring: Lymphocytes are multifunctional "drugs" that exhibit dynamic effects post-infusion. Upon encountering antigens, tumor-specific T cells activate and/ or release various proteins capable of triggering tumor killing, T cell proliferation, recruitment of other immune cells, or immune modulation. Therefore, measuring which proteins are secreted by which cells, in what quantities, and at what time points can provide profound insights into why specific patients respond or fail to respond, offering critical feedback for designing more effective trials. These trial systems will allow direct and meaningful comparisons of clinical approaches, thereby facilitating the design of rational next-generation therapeutic strategies.

For treatment, the amount administered is an amount effective to produce the desired effect. An effective amount may be provided in a single dose or a series of doses. An effective amount may be provided via bolus or by continuous infusion.

An "effective amount" (or "therapeutically effective amount") is an amount sufficient to elicit a beneficial or desired clinical result upon administration. An effective amount may be administered to a subject in one or more doses. In terms of therapy, an effective amount is an amount sufficient to alleviate, ameliorate, stabilize, reverse, slow, or delay the progression of the disease, or otherwise reduce the pathological consequences of the disease. The effective amount is generally determined by a physician based on the specific circumstances and falls within the skill of the art. When determining an appropriate dosage to achieve an effective amount, several factors are typically considered. These factors include the subject's age, sex, and weight; the condition being treated; the severity of the condition; and the dosage form and effective concentration of the immune effector cells administered.

The term "subject" refers to a mammal, such as a primate mammal, for example, a human. In some embodiments, the subject ( e.g., a human) suffers from a disease related to Ly6G6D.

The subject matter disclosed herein provides kits for treating or preventing cancer. In certain embodiments, the kit comprises a therapeutic or prophylactic composition comprising an effective amount of a unit dosage form of immune effector cells comprising a CAR targeting Ly6G6D. In specific embodiments, the cells further express at least one costimulatory ligand. In some embodiments, the kit comprises a sterile container containing a therapeutic or prophylactic vaccine; such containers may be boxes, ampoules, bottles, vials, tubes, bags, pouches, blisters, or other suitable container forms known in the art. Such containers may be made of plastic, glass, laminated paper, metal foil, or other materials suitable for containing pharmaceuticals.

The terms "cancer" and "cancerous" refer to or describe a physiological condition in mammals that is typically characterized by uncontrolled cell growth/proliferation. Aspects of cancer include solid tumor cancers and non-solid tumor cancers. Solid cancerous tumors include, but are not limited to, colorectal cancer, esophageal cancer, gastric cancer, small intestine cancer, large intestine cancer, or adenocarcinoma (e.g., colorectal adenocarcinoma, gastric adenocarcinoma, or pancreatic adenocarcinoma), or metastatic cancer thereof, such as metastatic colorectal adenocarcinoma. The cancer may be LY6G6D-positive cancer.

If desired, the immune effector cells are provided together with instructions for administering the cells to a subject having cancer or at risk of developing cancer. The instructions typically include information regarding the use of the composition for treating or preventing cancer. In other embodiments, the instructions include at least one of the following: description of the therapeutic agent; dosage regimen and method of administration for treating or preventing cancer or symptoms thereof; precautions; warnings; indications; contraindications; overdose information; adverse reactions; animal pharmacology; clinical studies; and/or references. The instructions may be printed directly on the container (when present), applied as a label to the container, or provided as a separate sheet, brochure, card, or folded printed matter within or with the container.

The embodiments of the present invention will be described in detail below with reference to the accompanying drawings and examples. However, those skilled in the art will understand that the following drawings and examples are for illustrative purposes only and do not limit the scope of the present invention. Various objects and advantageous aspects of the present invention will become apparent to those skilled in the art from the following detailed description of the accompanying drawings and preferred embodiments.

### Description of the drawings

Figure1 shows the results of CAR-T cell positivity rates determined in Example 2;
Figure 2 shows the *in vitro* cytotoxicity results of different CAR-T cells in Example 3;
Figure 3 shows the measurement results of cytokine release levels after co-incubation of different CAR-T cells (D10, D46, D59, D70, D72) with target cells in Example 4;
Figure 4 shows the measurement results of cytokine release levels after co-incubation of different CAR-T cells (D76, D79, D85, D103) with target cells in Example 4;
Figure 5 shows the in vivo anti-tumor results of different CAR-T cells in mice in Example 5.

### EXAMPLES

The scheme of the present invention will be explained below with reference to embodiments. Those skilled in the art will appreciate that the following embodiments are only used to illustrate the present invention and should not be construed as limiting the scope of the present invention. If the specific technology or conditions are not indicated in the examples, the technology or conditions described in the literature in the art or the product descriptions are performed. If the reagents or instruments used are not specified by the manufacturers, they are all conventional products that are commercially available.

### Example 1: Design and construction of CAR targeting Ly6G6D antigen, and lentiviral packaging

### 1.1 Construction of CAR targeting Ly6G6D antigen

The CAR structure targeting Ly6G6D includes: Signal peptide - scFv - hinge region (CD8 hinge) - CD8α transmembrane region (CD8TM) - ICOS - 4-1BB - CD3 ζ, wherein the CAR structures and their numbers are shown in Table 1.

**Table 1**

| Abbreviation | Structure |
|---|---|
| D9-CAR | Single peptide -D9 scFv -CD8hinge + CD8TM-ICOS-4-1BB-CD3ζ |
| D10-CAR | Single peptide -D10 scFv -CD8hinge + CD8TM-ICOS-4-1BB-CD3ζ |
| D28-CAR | Single peptide -D28 scFv -CD8hinge + CD8TM-ICOS-4-1BB-CD3ζ |
| D30-CAR | Single peptide -D30 scFv -CD8hinge + CD8TM-ICOS-4-1BB-CD3ζ |
| D32-CAR | Single peptide -D32 scFv -CD8hinge + CD8TM-ICOS-4-1BB-CD3ζ |
| D46-CAR | Single peptide -D46 scFv -CD8hinge + CD8TM-ICOS-4-1BB-CD3ζ |
| D59-CAR | Single peptide -D59 scFv -CD8hinge + CD8TM-ICOS-4-1BB-CD3ζ |
| D61-CAR | Single peptide -D61 scFv -CD8hinge + CD8TM-ICOS-4-1BB-CD3ζ |
| D70-CAR | Single peptide -D70 scFv -CD8hinge + CD8TM-ICOS-4-1BB-CD3ζ |
| D72-CAR | Single peptide -D72 scFv -CD8hinge + CD8TM-ICOS-4-1BB-CD3ζ |
| D76-CAR | Single peptide -D76 scFv -CD8hinge + CD8TM-ICOS-4-1BB-CD3ζ |
| D79-CAR | Single peptide -D79 scFv -CD8hinge + CD8TM-ICOS-4-1BB-CD3ζ |
| D85-CAR | Single peptide -D85 scFv -CD8hinge + CD8TM-ICOS-4-1BB-CD3ζ |
| D103-CAR | Single peptide -D103 scFv -CD8hinge + CD8TM-ICOS-4-1BB-CD3ζ |

The scFv targeting Ly6G6D has the amino acid sequence shown in any one of SEQ ID NO:81-90 or SEQ ID NO:152-155; wherein, the scFv of D9-CAR has the amino acid sequence shown in SEQ ID NO:81; the scFv of D10-CAR has the amino acid sequence shown in SEQ ID NO:82; the scFv of D28-CAR has the amino acid sequence shown in SEQ ID NO:83; the scFv of D30-CAR has the amino acid sequence shown in SEQ ID NO:84; the scFv of D32-CAR has the amino acid sequence shown in SEQ ID NO:85; the scFv of D46-CAR has the amino acid sequence shown in SEQ ID NO:86; the scFv of D59-CAR has the amino acid sequence shown in SEQ ID NO:87; the scFv of D61-CAR has the amino acid sequence shown in SEQ ID NO:88; the scFv of D70-CAR has the amino acid sequence shown in SEQ ID NO:89; the scFv of D72-CAR has the amino acid sequence shown in SEQ ID NO:90; the scFv of D76-CAR has the amino acid sequence shown in SEQ ID NO:152; the scFv of D79-CAR has the amino acid sequence shown in SEQ ID NO:153; the scFv of D85-CAR has the amino acid sequence shown in SEQ ID NO:154; the scFv of D103-CAR has the amino acid sequence shown in SEQ ID NO:155;

The CAR targeting Ly6G6D has the nucleotide (DNA) sequence shown in any one of SEQ ID NO:109-118 or SEQ ID NO:160-163; and wherein,
the signal peptide comprises the nucleotide sequence shown in SEQ ID NO:119:
   ATGGGCGTCAAGGTCCTGTTCGCCCTGATCTGCATCGCCGTCGCCGAGGCC (SEQ ID NO: 119);
the EcoRI restriction site comprises the nucleotide sequence GAATTC;
the hinge region comprises the nucleotide sequence shown in SEQ ID NO:120: 
ACCACGACGCCAGCGCCGCGACCACCAACACCGGCGCCCACCATCGCGTCGCAGC
CCCTGTCCCTGCGCCCAGAGGCGTGCCGGCCAGCGGCGGGGGCGCAGTGCACACGAG
GGGGCTGGACTTCGCCTGTGATATCTAC (SEQ ID NO: 120);
the transmembrane domain of the CD8α protein comprises the nucleotide sequence shown in SEQ ID NO:121: 
ATCTGGGCGCCCTTGGCCGGGACTTGTGGGGTCCTTCTCCTGTCACTGGTTATCACC
CTTTACTGC (SEQ ID NO: 121);
the ICOS domain comprises the nucleotide sequence shown in SEQ ID NO:122: 
TGTTGGCTTACAAAAAAGAAGTATTCATCCAGTGTGCACGACCCTAACGGTGAATA
CATGTTCATGAGAGCAGTGAACACAGCCAAAAAATCTAGACTCACAGATGTGACCCTA
(SEQ ID NO: 122);
the 4-1BB domain comprises the nucleotide sequence shown in SEQ ID NO:123: 
AAGCGCGGCCGCAAGAAGCTGCTGTACATCTTCAAGCAGCCCTTCATGCGCCCCGT
GCAGACCACCCAGGAGGAGGACGGCTGTAGCTGCCGCTTCCCCGAGGAGGAGGAGGG
CGGCTGCGAGCTG (SEQ ID NO: 123);
the signaling domain of CD3ζ comprises the nucleotide sequence shown in SEQ ID NO:124: 
CGCGTGAAATTTAGCCGCAGCGCCGACGCCCCCGCCTACCAGCAGGGCCAGAACC
AGCTGTACAACGAGCTGAACCTGGGCCGCCGCGAGGAGTACGACGTGCTGGACAAGCG
CCGgGGCCGCGACCCCGAGATGGGCGGCAAGCCCCAGCGCCGCAAGAACCCCCAGGAG
GGCCTGTACAACGAGCTGCAGAAGGACAAGATGGCCGAaGCCTACAGCGAGATCGGCA
TGAAGGGCGAGCGCCGCCGgGGCAAGGGCCACGACGGCCTGTACCAGGGCCTGAGCAC
CGCCACCAAGGACACCTACGACGCCCTGCACATGCAGGCCCTGCCCCCCCGC (SEQ ID
NO: 124);
 The DNA sequence of the aforementioned CAR was artificially synthesized and constructed into a fourth-generation lentiviral shuttle plasmid.

### 1.2 Lentiviral packaging of CAR vectors targeting Ly6G6D based on different scFvs

a. 2 * 10⁷ 293T cells were seeded into a 15 cm dish containing 25 mL of cell growth medium and placed in an incubator at 37 °C with 5% CO₂ to culture overnight for later use.
b. The lentivirus packaging system was prepared according to Table 2 below:

**Table 2**

| Component | Volume |
|---|---|
| Solution A | |
| Opti-MEM Reduced Serum Medium | 3 mL |
| Lipofectamine 3000 Transfection Reagent | 126 µL |
| Solution B | |
| Opti-MEM Reduced Serum Medium | 3 mL |
| P3000 Enhancer Reagent | 108 µL |
| Mixture of three helper plasmids | 12.8 µg each |
| CAR-expressing shuttle plasmid | 12.8 µg |

Solution A and Solution B were each mixed separately. Then, all of Solution A was transferred into Solution B. After thorough mixing, the mixture was left to stand at room temperature for 15 minutes. Subsequently, 10 mL of lentivirus transfection medium was added and mixed.
c. The culture medium of the 293T cells in the 15 cm dish was removed. The entire transfection system described above was added to the dish. After gentle mixing, the cells were placed in an incubator at 37 °C with 5% CO₂. Six hours after transfection, the transfection system was aspirated, and 30 mL of lentivirus packaging medium was added. After continued culture for 48 hours, the culture supernatant was collected, concentrated or purified, and then stored in an ultra-low temperature freezer for later use.

### Example 2: Preparation of CAR-T Cells and Determination of Positive Rate

CAR-T cell preparation: T cells were thawed, resuspended in CTS complete medium (containing serum substitute, IL-7, and IL-15), and activated using anti-CD3/CD28 activation beads. Three days after activation, the activation beads were removed, and the T cells were subjected to lentiviral transduction. Twenty-four hours after infection, the cell culture medium was replaced to remove residual lentivirus. After infection, the detection of CAR positive rate was initiated 2 days after culture using a flow cytometer.

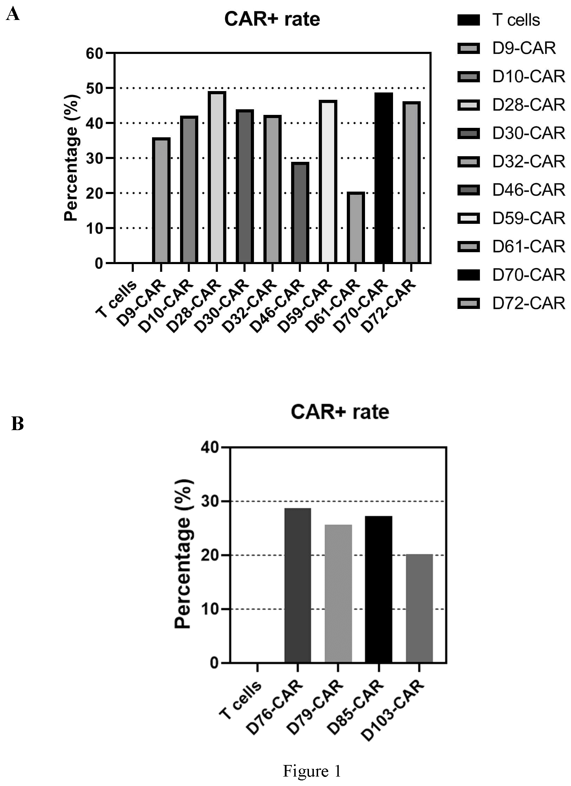

The experimental results are shown in Figure 1. As indicated in Figures 1A and 1B, D9-CAR, D10-CAR, D28-CAR, D30-CAR, D32-CAR, D46-CAR, D59-CAR, D61-CAR, D70-CAR, D72-CAR, D85-CAR, D103-CAR, D76-CAR, and D79-CAR were all effectively expressed on the surface of CAR-T cells. The positive rates varied among different CAR molecules: the expression rates of D61-CAR and D103-CAR were approximately 20%; D79-CAR was approximately 25%; D46-CAR, D76-CAR, and D85-CAR were approximately 30%; D9-CAR was approximately 35%; and seven CAR molecules, including D10-CAR, D28-CAR, D30-CAR, D32-CAR, D59-CAR, D70-CAR, and D72-CAR, exhibited expression rates between 40% and 50%.

### Example 3: Assessment of Cytotoxic Effects of Different CAR-T Cells

Target cell plating: Target cells in the logarithmic growth phase were collected, centrifuged at 500 g for 4 minutes, and the supernatant was discarded. The cells were then resuspended in CTS complete medium to a density of 2 × 10⁶ cells/mL and seeded into a flat-bottom 96-well plate at 100 µL per well, resulting in 2 * 10⁴ cells per well.

Co-incubation of effector cells and target cells: After using T cells to adjust the positive rate to be consistent for each group, plating was performed according to the set effector-to-target ratios. In addition, for each effector-to-target ratio, a CAR-T cell-only group was set as a negative control for effector cells; a target cell-only group was also set as a negative control; and a target cell + lysis buffer group was set as a positive control for target cell lysis. After 20-22 hours of co-incubation, the cell culture supernatant was collected, and the killing ability of CAR-T cells was detected using the LDH method.

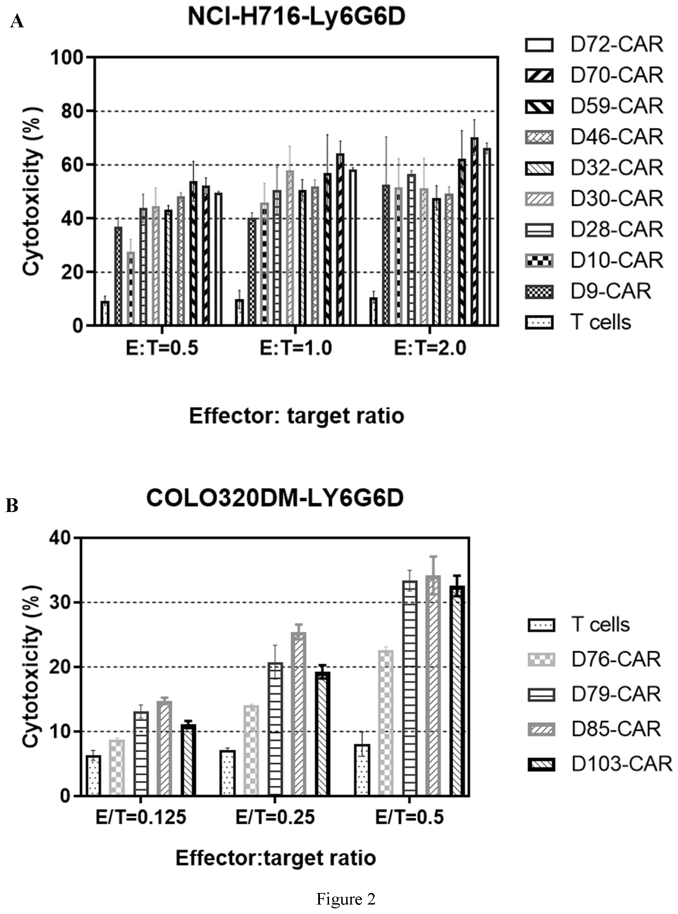

The experimental results are shown in Figure 2. As shown in Figure 2A, D9-CAR-T, D10-CAR-T, D28-CAR-T, D30-CAR-T, D32-CAR-T, D46-CAR-T, D59-CAR-T, D70-CAR-T, and D72-CAR-T all effectively killed target cells at effector-to-target ratios of 0.5:1, 1:1, and 2:1. Among them, D59-CAR-T, D70-CAR-T, and D72-CAR-T exhibited the strongest cytotoxicity against target cells at an effector-to-target ratio of 2.0, with killing efficiencies of approximately 62% for D59-CAR-T, 70% for D70-CAR-T, and 66% for D72-CAR-T. As shown in Figure 2B, D76-CAR-T, D79-CAR-T, D85-CAR-T, and D103-CAR-T all effectively killed target cells at effector-to-target ratios of 1:8, 1:4, and 1:2 in a dose-dependent manner. Among these, D79-CAR-T and D85-CAR-T cells demonstrated relatively better efficacy.

### Example 4: Determination of Cytokine Release Levels After Co-incubation of CAR-T Cells Based on Different scFvs with Target Cells

CAR-T cells were co-incubated with target cells according to the method described in Example 3. The first batch of CAR-T cells, including D10-CAR-T, D46-CAR-T, D59-CAR-T, D70-CAR-T, and D72-CAR-T, was co-incubated with the NCI-H716-Ly6G6D stable cell line expressing Ly6G6D. The second batch of CAR-T cells, including D76, D79, D85, and D103 CAR-T cells, was co-incubated with the colo320DM-Ly6G6D stable cell line expressing Ly6G6D. Control groups included cells incubated with CAR-T cells alone (without target cells) and cells incubated with target cells alone. Cell supernatants were collected simultaneously with cytotoxicity assays and diluted 5-fold for subsequent use. The release levels of six cytokines, including IL-2, IL-4, IL-6, IL-10, TNF-α, and IFN-γ, in the samples were detected using the BD Cytometric Bead Array Human Th1/Th2 Cytokine Kit II (Catalog No. 551809).

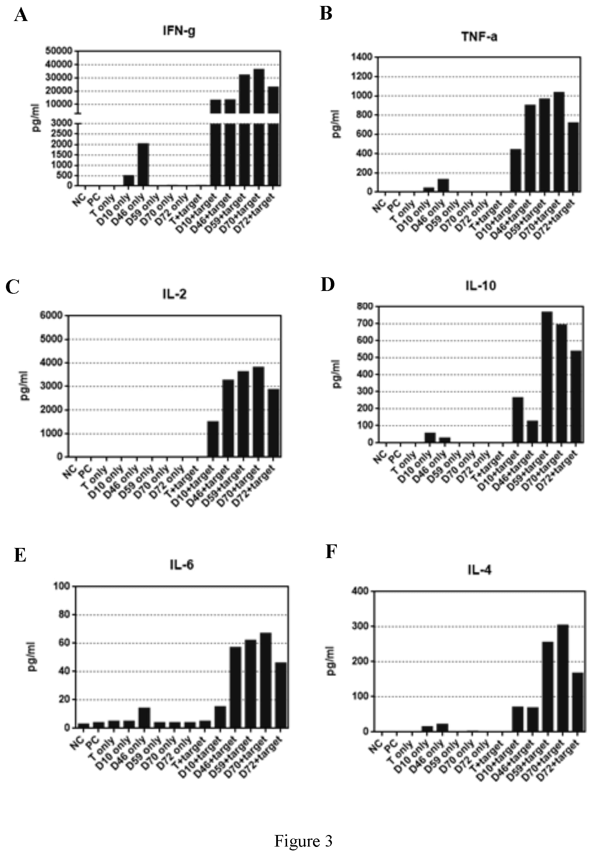

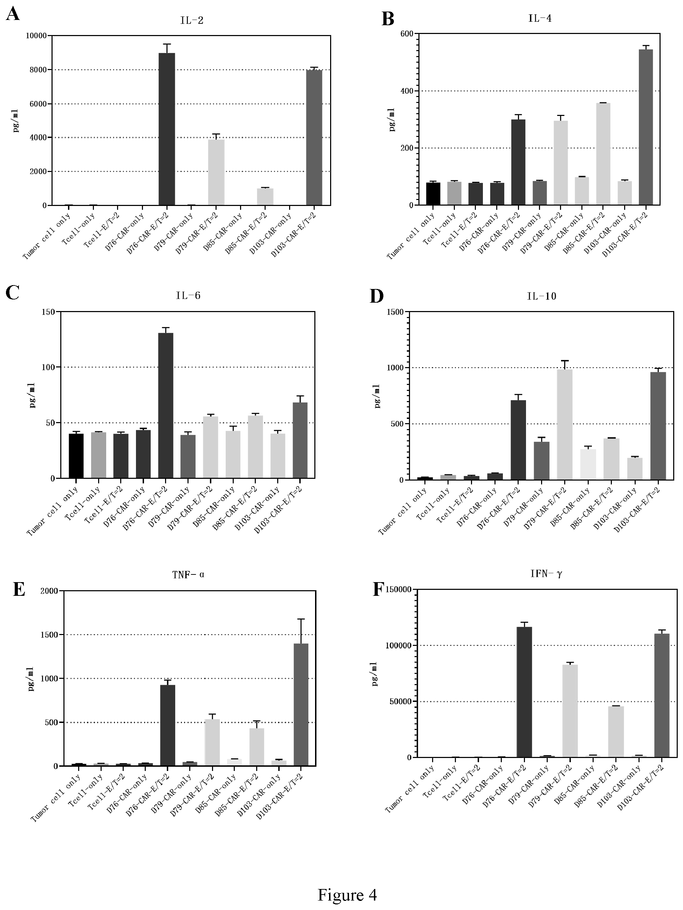

The experimental results are shown in Figures 3 and 4. In Figure 3, D10, D46, D59, D70, and D72 refer to D10-CAR-T, D46-CAR-T, D59-CAR-T, D70-CAR-T, and D72-CAR-T, respectively; the figure displays the cytokine release levels for each CAR-T cell group and the control groups. Figure 4 shows the cytokine release levels for D76, D79, D85, and D103 CAR-T cells and their respective control groups. The abscissa indicates the names of the various groups, and the ordinate indicates the concentration of the relevant factors, with units of pg/mL;

As shown in Figure 3, after co-incubation with target cells, D10-CAR-T, D46-CAR-T, D59-CAR-T, D70-CAR-T, and D72-CAR-T cells effectively released cytokines such as IFN-γ, TNF-α, and IL-2, which promote CAR-T cell-mediated killing of target cells and T cell proliferation. Cytokines including IL-4, IL-6, and IL-10 were also released to varying degrees. The levels of cytokine release differed among the various CAR-T cells after co-incubation with target cells; among them, D70-CAR-T cells exhibited the highest release levels of IFN-γ, TNF-α, and IL-2, reaching approximately 40,000 pg/mL, over 1,000 pg/mL, and nearly 4,000 pg/mL, respectively. D59-CAR-T cells showed the second-highest release levels, reaching 30,000 pg/mL, nearly 1,000 pg/mL, and nearly 4,000 pg/mL, respectively. Under conditions without co-incubation with target cells, D10-CAR-T and D46-CAR-T cells exhibited high levels of spontaneous release of IFN-γ and TNF-α; among them, D46-CAR-T cells showed the highest spontaneous release levels, with IFN-γ spontaneous release at approximately 2,000 pg/mL and TNF-α spontaneous release exceeding 100 pg/mL. In contrast, D59-CAR-T, D70-CAR-T, and D72-CAR-T cells showed no significant spontaneous release of IFN-γ or TNF-α. No significant spontaneous release of IL-2 was observed in any group.

As shown in Figure 4: (1) After co-incubation with colo320DM-Ly6G6D cells, D76, D79, D85, and D103 CAR-T cells effectively released factors such as IL-2, IFN-γ, and TNF-α; among them, D76 CAR-T cells exhibited the highest release, while D85 CAR-T cells exhibited the lowest. CAR-T cells not co-incubated with target cells showed no significant release. (2) After co-incubation with target cells, all four types of CAR-T cells significantly released IL-4, with the D103 group showing the highest level. (3) Only D76 CAR-T cells significantly released IL-6 after co-incubation with target cells. (4) D76 CAR-T cells exhibited low baseline release levels of IL-10, which increased significantly after co-incubation with target cells. D79, D85, and D103 CAR-T cells all exhibited high levels of baseline IL-10 release, and their release levels further increased after co-incubation with target cells.

### Example 5: In Vivo Evaluation of Antitumor Efficacy of Preferred CAR-T Cells Based on Different scFvs

### Preparation of CAR- T Cells:

CAR-T cell preparation: T cells were thawed, resuspended in CTS complete medium, and activated using anti-CD3/CD28 activation beads. Three days after activation, the activation beads were removed, and the T cells were subjected to lentiviral transduction. Twenty-four hours after infection, the cell culture medium was replaced to remove residual lentivirus. After infection, the detection of CAR positive rate was initiated 2 days after culture using a flow cytometer. Before cell cryopreservation, the CAR-positive rate of CAR-T cells in each group was adjusted to a consistent level using uninfected T cells. The cells were then resuspended in cryopreservation medium, aliquoted, and transferred to a programmed freezer before being placed in an ultra-low temperature freezer. After one day, they were transferred to liquid nitrogen for long-term storage.

Thawing and administration: After thawing, the cells were resuspended in pre-chilled PBS before administration. In the first batch of experiments, CAR-T cell groups D10, D46, D59, D70, and D72 were administered at a dose of 3.6 * 10⁶ CAR+ cells per mouse; in the second batch of experiments, CAR-T cell groups D79, D85, and D103 were administered at a dose of 2.0 * 10⁶ CAR+ cells per mouse. The administration volume for each mouse was 200 µL. When the tumor volume reached approximately 150 mm³, CAR-T cells were infused via the tail vein. Tumor volumes were measured twice a week after infusion.

### Data Processing:

$Tumor Volume \left({mm}^{3}\right) = Length \left(mm\right) * Width \left(mm\right) * Width \left(mm\right) * 0.5$ 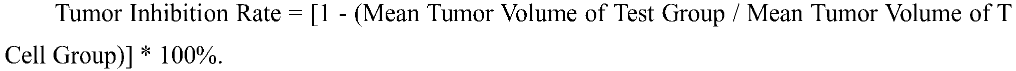 Tumor Inhibition Rate = [1 - (Mean Tumor Volume of Test Group / Mean Tumor Volume of T Cell Group)] * 100%.

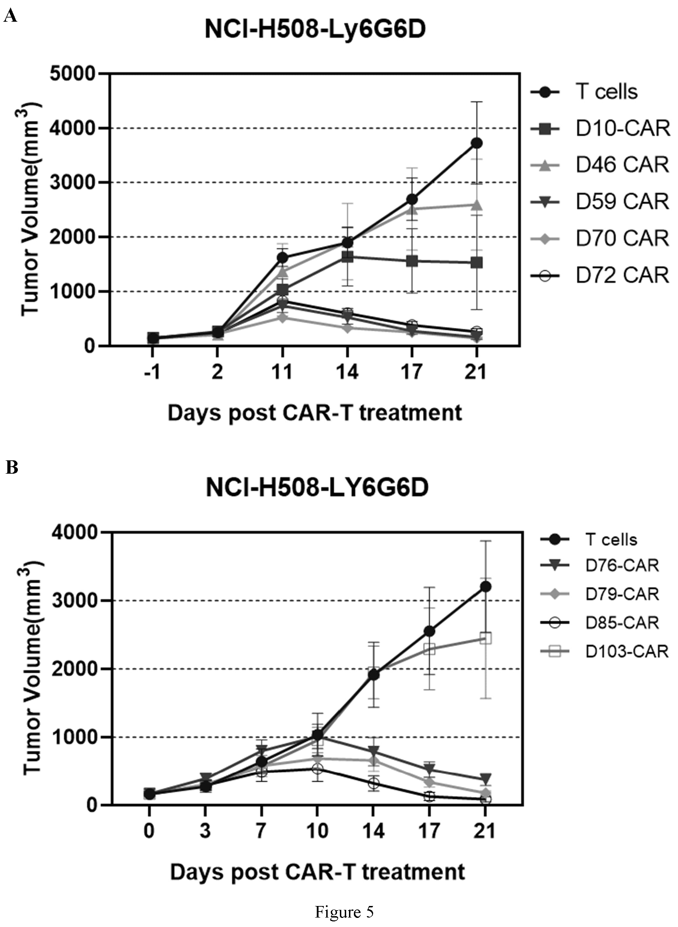

The experimental results are shown in Figure 5. As indicated by the processed data in Figure 5A, all five CAR-T cell groups (D10-CAR, D46-CAR, D59-CAR, D70-CAR, and D72-CAR) demonstrated tumor inhibitory effects compared to the T cell group. The inhibitory effects of different CAR-T cell groups on NCI-H508-Ly6G6D tumors varied: D46-CAR-T showed an inhibition rate of 30.5%, and D10-CAR-T showed an inhibition rate of 59%. At 21 days post-CAR-T infusion, the tumor inhibition rates for D59-CAR-T, D70-CAR-T, and D72-CAR-T reached 95.4%, 96%, and 92.9%, respectively.

In another batch of animal experiments, data processing yielded the results shown in Figure 5B. At 21 days post-infusion, based on mean tumor volume calculations, the D76-CAR, D79-CAR, D85-CAR, and D103-CAR groups all demonstrated tumor inhibitory effects compared to the T cell group. The inhibitory effects varied among the different CAR-T cell groups: D76-CAR-T showed an inhibition rate of 88.34%, D79-CAR-T showed 94.46%, D85-CAR-T showed 97.28%, and D103-CAR-T showed 33.68%. Among these, D85-CAR-T cells exhibited a relatively superior inhibitory effect.

Reference throughout this specification to "an embodiment," "some embodiments," "one embodiment", "another example," "an example," "a specific example," or "some examples," means that a particular feature, structure, material, or characteristic described in connection with the embodiment or example is included in at least one embodiment or example of the present disclosure. Thus, the appearances of the above terms throughout this specification are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although embodiments of the present invention have been shown and described above, it is understood that the aforementioned embodiments are exemplary and should not be construed as limiting the present invention. Those of ordinary skill in the art may make changes, modifications, substitutions, and variations to the aforementioned embodiments within the scope of the present invention.

## Claims

1. A chimeric antigen receptor targeting Ly6G6D, **characterized in that** the chimeric antigen receptor comprises an extracellular antigen-binding domain that binds to Ly6G6D, a transmembrane domain and an intracellular domain, wherein the extracellular antigen-binding domain comprises an antibody or antigen-binding fragment that targets and binds to Ly6G6D, wherein the antibody or antigen-binding fragment comprises a heavy chain variable region, the heavy chain variable region comprises complementarity determining regions: H-CDR1, H-CDR2, and H-CDR3; wherein,
H-CDR1 has the amino acid sequence shown in SEQ ID NO: 1, SEQ ID NO: 7, SEQ ID NO: 13, SEQ ID NO: 18, SEQ ID NO: 22, SEQ ID NO: 28, SEQ ID NO: 34, SEQ ID NO: 40, SEQ ID NO: 46, SEQ ID NO: 50, SEQ ID NO: 127 or SEQ ID NO: 138; and
H-CDR2 has the amino acid sequence shown in SEQ ID NO: 2, SEQ ID NO: 8, SEQ ID NO: 14, SEQ ID NO: 19, SEQ ID NO: 23, SEQ ID NO: 29, SEQ ID NO: 35, SEQ ID NO: 41, SEQ ID NO: 47, SEQ ID NO: 51 or SEQ ID NO: 139; and
H-CDR3 has the amino acid sequence shown in SEQ ID NO: 3, SEQ ID NO: 9, SEQ ID NO: 15, SEQ ID NO: 20, SEQ ID NO: 24, SEQ ID NO: 30, SEQ ID NO: 36, SEQ ID NO: 42, SEQ ID NO: 48, SEQ ID NO: 52, SEQ ID NO: 128, SEQ ID NO: 132 or SEQ ID NO: 140;
the antibody or antigen-binding fragment comprises a light chain variable region, the light chain variable region comprises complementarity determining regions: L-CDR1, L-CDR2, and L-CDR3; wherein,
L-CDR1 has the amino acid sequence shown in SEQ ID NO: 4, SEQ ID NO: 10, SEQ ID NO: 25, SEQ ID NO: 31, SEQ ID NO: 37, SEQ ID NO: 43, SEQ ID NO: 53, SEQ ID NO: 129, SEQ ID NO: 135 or SEQ ID NO: 141; and
L-CDR2 has the amino acid sequence shown in SEQ ID NO: 5, SEQ ID NO: 11, SEQ ID NO: 16, SEQ ID NO: 26, SEQ ID NO: 32, SEQ ID NO: 38, SEQ ID NO: 44, SEQ ID NO: 54, SEQ ID NO: 130, SEQ ID NO: 133, SEQ ID NO: 136 or SEQ ID NO: 142; and
L-CDR3 has the amino acid sequence shown in SEQ ID NO: 6, SEQ ID NO: 12, SEQ ID NO: 17, SEQ ID NO: 21, SEQ ID NO: 27, SEQ ID NO: 33, SEQ ID NO: 39, SEQ ID NO: 45, SEQ ID NO: 49, SEQ ID NO: 131, SEQ ID NO: 134, SEQ ID NO: 137 or SEQ ID NO: 143.

2. The chimeric antigen receptor according to claim 1, **characterized in that** complementarity determining regions of the heavy chain variable region: H-CDR1, H-CDR2 and H-CDR3 are selected from any one of the following groups:
(1) H-CDR1 has the amino acid sequence shown in SEQ ID NO:1, H-CDR2 has the amino acid sequence shown in SEQ ID NO:2 and H-CDR3 has the amino acid sequence shown in SEQ ID NO:3; or
(2) H-CDR1 has the amino acid sequence shown in SEQ ID NO:7, H-CDR2 has the amino acid sequence shown in SEQ ID NO:8 and H-CDR3 has the amino acid sequence shown in SEQ ID NO:9; or
(3) H-CDR1 has the amino acid sequence shown in SEQ ID NO:13, H-CDR2 has the amino acid sequence shown in SEQ ID NO:14 and H-CDR3 has the amino acid sequence shown in SEQ ID NO:15; or
(4) H-CDR1 has the amino acid sequence shown in SEQ ID NO:18, H-CDR2 has the amino acid sequence shown in SEQ ID NO:19 and H-CDR3 has the amino acid sequence shown in SEQ ID NO:20; or
(5) H-CDR1 has the amino acid sequence shown in SEQ ID NO:22, H-CDR2 has the amino acid sequence shown in SEQ ID NO:23 and H-CDR3 has the amino acid sequence shown in SEQ ID NO:24; or
(6) H-CDR1 has the amino acid sequence shown in SEQ ID NO:28, H-CDR2 has the amino acid sequence shown in SEQ ID NO:29 and H-CDR3 has the amino acid sequence shown in SEQ ID NO:30; or
(7) H-CDR1 has the amino acid sequence shown in SEQ ID NO:34, H-CDR2 has the amino acid sequence shown in SEQ ID NO:35 and H-CDR3 has the amino acid sequence shown in SEQ ID NO:36; or
(8) H-CDR1 has the amino acid sequence shown in SEQ ID NO:40, H-CDR2 has the amino acid sequence shown in SEQ ID NO:41 and H-CDR3 has the amino acid sequence shown in SEQ ID NO:42; or
(9) H-CDR1 has the amino acid sequence shown in SEQ ID NO:46, H-CDR2 has the amino acid sequence shown in SEQ ID NO:47 and H-CDR3 has the amino acid sequence shown in SEQ ID NO:48; or
(10) H-CDR1 has the amino acid sequence shown in SEQ ID NO:50, H-CDR2 has the amino acid sequence shown in SEQ ID NO:51 and H-CDR3 has the amino acid sequence shown in SEQ ID NO:52 or
(11) H-CDR1 has the amino acid sequence shown in SEQ ID NO:127, H-CDR2 has the amino acid sequence shown in SEQ ID NO:29 and H-CDR3 has the amino acid sequence shown in SEQ ID NO:128; or
(12) H-CDR1 has the amino acid sequence shown in SEQ ID NO:18, H-CDR2 has the amino acid sequence shown in SEQ ID NO:29 and H-CDR3 has the amino acid sequence shown in SEQ ID NO:132; or
(13) H-CDR1 has the amino acid sequence shown in SEQ ID NO:18, H-CDR2 has the amino acid sequence shown in SEQ ID NO:29 and H-CDR3 has the amino acid sequence shown in SEQ ID NO:128; or
(14) H-CDR1 has the amino acid sequence shown in SEQ ID NO:138, H-CDR2 has the amino acid sequence shown in SEQ ID NO:139 and H-CDR3 has the amino acid sequence shown in SEQ ID NO: 140.

3. The chimeric antigen receptor according to claim 1, **characterized in that** the complementarity determining regions of the light chain variable region: H-CDR1, H-CDR2 and H-CDR3 are selected from any one of the following groups:
(1) L-CDR1 has the amino acid sequence shown in SEQ ID NO:4, L-CDR2 has the amino acid sequence shown in SEQ ID NO:5 and L-CDR3 has the amino acid sequence shown in SEQ ID NO:6; or
(2) L-CDR1 has the amino acid sequence shown in SEQ ID NO:10, L-CDR2 has the amino acid sequence shown in SEQ ID NO:11 and L-CDR3 has the amino acid sequence shown in SEQ ID NO:12; or
(3) L-CDR1 has the amino acid sequence shown in SEQ ID NO:4, L-CDR2 has the amino acid sequence shown in SEQ ID NO:16 and L-CDR3 has the amino acid sequence shown in SEQ ID NO:17; or
(4) L-CDR1 has the amino acid sequence shown in SEQ ID NO:4, L-CDR2 has the amino acid sequence shown in SEQ ID NO:16 and L-CDR3 has the amino acid sequence shown in SEQ ID NO:21; or
(5) L-CDR1 has the amino acid sequence shown in SEQ ID NO:25, L-CDR2 has the amino acid sequence shown in SEQ ID NO:26 and L-CDR3 has the amino acid sequence shown in SEQ ID NO:27; or
(6) L-CDR1 has the amino acid sequence shown in SEQ ID NO:31, L-CDR2 has the amino acid sequence shown in SEQ ID NO:32 and L-CDR3 has the amino acid sequence shown in SEQ ID NO:33; or
(7) L-CDR1 has the amino acid sequence shown in SEQ ID NO:37, L-CDR2 has the amino acid sequence shown in SEQ ID NO:38 and L-CDR3 has the amino acid sequence shown in SEQ ID NO:39; or
(8) L-CDR1 has the amino acid sequence shown in SEQ ID NO:43, L-CDR2 has the amino acid sequence shown in SEQ ID NO:44 and L-CDR3 has the amino acid sequence shown in SEQ ID NO:45; or
(9) L-CDR1 has the amino acid sequence shown in SEQ ID NO:37, L-CDR2 has the amino acid sequence shown in SEQ ID NO:38 and L-CDR3 has the amino acid sequence shown in SEQ ID NO:49; or
(10) L-CDR1 has the amino acid sequence shown in SEQ ID NO:53, L-CDR2 has the amino acid sequence shown in SEQ ID NO:54 and L-CDR3 has the amino acid sequence shown in SEQ ID NO:45; or
(11) L-CDR1 has the amino acid sequence shown in SEQ ID NO:129, L-CDR2 has the amino acid sequence shown in SEQ ID NO:130 and L-CDR3 has the amino acid sequence shown in SEQ ID NO:131; or
(12) L-CDR1 has the amino acid sequence shown in SEQ ID NO:37, L-CDR2 has the amino acid sequence shown in SEQ ID NO:133 and L-CDR3 has the amino acid sequence shown in SEQ ID NO:134; or
(13) L-CDR1 has the amino acid sequence shown in SEQ ID NO:135, L-CDR2 has the amino acid sequence shown in SEQ ID NO:136 and L-CDR3 has the amino acid sequence shown in SEQ ID NO:137; or
(14) L-CDR1 has the amino acid sequence shown in SEQ ID NO:141, L-CDR2 has the amino acid sequence shown in SEQ ID NO:142 and L-CDR3 has the amino acid sequence shown in SEQ ID NO: 143.

4. The chimeric antigen receptor according to any one of claims 1-3, **characterized in that** the antibody or antigen-binding fragment comprises a heavy chain variable region framework sequence and a light chain variable region framework sequence, wherein the framework sequence is derived from a murine antibody, a human antibody, a primate-derived antibody, or mutants thereof.

5. The chimeric antigen receptor according to any one of claims 1-4, **characterized in that** the heavy chain variable region of the antibody or antigen-binding fragment comprises an amino acid sequence having at least 85%, at least 90%, at least 95%, at least 96%, at least 97%, at least 98%, or at least 99% sequence identity to any one of SEQ ID NO:55-64 or SEQ ID NO:144-147.

6. The chimeric antigen receptor according to any one of claims 1-5, **characterized in that** the light chain variable region of the antibody or antigen-binding fragment comprises an amino acid sequence having at least 85%, at least 90%, at least 95%, at least 96%, at least 97%, at least 98%, or at least 99% sequence identity to any one of SEQ ID NO:65-76 or SEQ ID NO:148-151.

7. The chimeric antigen receptor according to any one of claims 1-6, **characterized in that** the heavy chain variable region and the light chain variable region of the extracellular antigen-binding domain are connected via a linker.

8. The chimeric antigen receptor according to any one of claims 1-7, **characterized in that** the linker comprises the amino acid sequence shown in any one of SEQ ID NO: 77-80.

9. The chimeric antigen receptor according to any one of claims 1-8, **characterized in that** the extracellular antigen-binding domain is a single-chain antibody.

10. The chimeric antigen receptor according to any one of claims 1-9, **characterized in that** the extracellular antigen-binding domain comprises an amino acid sequence having at least 85%, at least 90%, at least 95%, at least 96%, at least 97%, at least 98%, or at least 99% sequence identity to any one of SEQ ID NO:81-90 or SEQ ID NO:152-155.

11. The chimeric antigen receptor according to any one of claims 1-10, **characterized in that** the transmembrane domain comprises a transmembrane domain derived from a protein selected from the group consisting of: the α, β, or ζ chain of a T cell receptor, CD28, CD3ζ, CD45, CD4, CD5, CD8α, CD9, CD16, CD22, CD33, CD37, CD64, CD80, CD86, CD134, 4-1BB, CD154, or combinations thereof.

12. The chimeric antigen receptor according to any one of claims 1-11, **characterized in that** the transmembrane domain comprises a transmembrane domain derived from the CD8α protein.

13. The chimeric antigen receptor according to any one of claims 1-12, **characterized in that** the transmembrane domain comprises the amino acid sequence shown in SEQ ID NO:92.

14. The chimeric antigen receptor according to any one of claims 1-13, **characterized in that** the chimeric antigen receptor further comprises a hinge region.

15. The chimeric antigen receptor according to any one of claims 1-14, **characterized in that** the hinge region connects the extracellular antigen-binding domain and the transmembrane domain.

16. The chimeric antigen receptor according to any one of claims 1-15, **characterized in that** the hinge region comprises the amino acid sequence shown in SEQ ID NO:93.

17. The chimeric antigen receptor according to any one of claims 1-16, **characterized in that** the intracellular domain of the chimeric antigen receptor comprises a signaling domain derived from CD3ζ.

18. The chimeric antigen receptor according to any one of claims 1-17, **characterized in that** the intracellular domain of the chimeric antigen receptor comprises the amino acid sequence shown in SEQ ID NO:94.

19. The chimeric antigen receptor according to any one of claims 1-18, **characterized in that** the intracellular domain further comprises at least one costimulatory domain.

20. The chimeric antigen receptor according to any one of claims 1-19, **characterized in that** the costimulatory domain comprises a costimulatory domain derived from a protein selected from the group consisting of: CD28, 4-1BB, OX-40, ICOS, or combinations thereof.

21. The chimeric antigen receptor according to any one of claims 1-20, **characterized in that** the costimulatory domain comprises a domain derived from 4-1BB.

22. The chimeric antigen receptor according to claim 21, **characterized in that** the costimulatory domain comprises the amino acid sequence shown in SEQ ID NO:95.

23. The chimeric antigen receptor according to any one of claims 1-20, **characterized in that** the costimulatory domain comprises a domain derived from ICOS.

24. The chimeric antigen receptor according to claim 23, **characterized in that** the costimulatory domain comprises the amino acid sequence shown in SEQ ID NO:96.

25. The chimeric antigen receptor according to any one of claims 1-20, **characterized in that** the costimulatory domain comprises a domain derived from a combination of 4-1BB and ICOS.

26. The chimeric antigen receptor according to claim 25, **characterized in that** the costimulatory domain comprises the amino acid sequence shown in SEQ ID NO:97.

27. The chimeric antigen receptor according to any one of claims 1-26, **characterized in that** the intracellular domain comprises the amino acid sequence shown in SEQ ID NO:98.

28. The chimeric antigen receptor according to any one of claims 1-27, **characterized in that** the chimeric antigen receptor comprises an amino acid sequence shown in any one of SEQ ID NO: 99-108 or SEQ ID NO: 156-159.

29. The chimeric antigen receptor according to any one of claims 1-28, **characterized in that** the chimeric antigen receptor further comprises a signal peptide covalently linked to the N-terminus of the extracellular antigen-binding domain.

30. The chimeric antigen receptor according to any one of claims 1-29, **characterized in that** the signal peptide comprises the amino acid sequence shown in SEQ ID NO:91.

31. An isolated nucleic acid molecule, **characterized in that** the nucleic acid molecule comprises a nucleotide sequence encoding the chimeric antigen receptor to any one of claims 1-30.

32. The nucleic acid molecule according to claim 31, **characterized in that** the nucleic acid molecule comprises the amino acid sequence shown in any one of SEQ ID NO: 109-118 or SEQ ID NO: 160-163.

33. An expression vector, **characterized in that** the expression vector carries the nucleic acid molecule of claim 31 or 32.

34. The expression vector according to claim 33, **characterized in that** the expression vector is a γ-retroviral vector or a lentiviral vector.

35. An immune effector cell, **characterized in that** the immune effector cell comprises the chimeric antigen receptor to any one of claims 1-30, the nucleic acid molecule of claim 31 or 32, or the expression vector of claim 33 or 34.

36. The immune effector cell according to claim 35, **characterized in that** the immune effector cell is selected from T cells, natural killer cells, cytotoxic T lymphocytes, regulatory T cells, human embryonic stem cells, lymphoid progenitor cells, T cell precursor cells, or pluripotent stem cells capable of differentiating into lymphocytes, macrophages, B cells, and immune cells derived from induced pluripotent stem cells that have cell-killing capability.

37. A method for preparing an immune effector cell, **characterized in that** the method comprises introducing the expression vector of claim 33 or 34 into an immune effector cell.

38. A composition, **characterized in that** the composition comprises the immune effector cell of claim 35 or 36 and a pharmaceutically acceptable carrier.

39. A use of the chimeric antigen receptor according to any one of claims 1-30, the nucleic acid molecule according to claim 31 or 32, the expression vector according to claim 33 or 34, the immune effector cell according to claim 35 or 36, or the composition according to claim 38 in the preparation of a medicament, wherein the medicament is used for treating a disease or disorder associated with the expression of Ly6G6D; and the disease or disorder associated with the expression of Ly6G6D is colorectal cancer.

40. A kit for treating cancer, **characterized in that** the kit comprises the immune effector cell of claim 35 or 36.

41. The kit according to claim 40, **characterized in that** the kit further comprises instructions for using the immune effector cell to treat a cancer subject.

42. The kit according to claim 41, **characterized in that** the cancer is colorectal cancer.
